# EUROPEAN PATENT APPLICATION

(11) **EP 3 974 531 A1**
(43) Date of publication of application: **30.03.2022**
(21) Application number: 20809923.4
(22) Date of filing: 20.05.2020
(51) Int. Cl.: C12N 15/113, C12N 5/0783, A61K 31/7088, A61K 35/17, A61P 35/00

(54) **ASYMMETRIC SIRNA INHIBITING EXPRESSION OF PD-1**

(30) Priority: 20.05.2019 KR 20190058805
(71) Applicant: Olix Pharmaceuticals, Inc., Suwon-si, Gyeonggi-do 16226 (KR)
(72) Inventor: LEE, Dong Ki, Seoul 06600 (KR); KANG, Younggue, Suwon-si, Gyeonggi-do 16435 (KR); DUA, Pooja, Yongin-si, Gyeonggi-do 16928 (KR)
(74) Representative: Weickmann & Weickmann PartmbB
(86) International application number: PCT/KR2020/006610
(87) International publication number: WO 2020/235936

(57) **Abstract**

The present invention relates to an asymmetric siRNA that may inhibit expression of programmed cell death protein 1 (PD-1), and a use thereof, and more specifically, to: an asymmetric siRNA including an antisense strand including a sequence complementary to mRNA encoding PD-1, and a sense strand forming complementary bonds with the antisense strand; a pharmaceutical composition for preventing or treating cancer, including the asymmetric siRNA; an immune cell in which expression of PD-1 is inhibited, obtained by treatment with the siRNA; and an immune cell therapeutic agent for treating cancer, including the immune cell.

## Description

### TECHNICAL FIELD

The present invention relates to an asymmetric siRNA that may inhibit expression of programmed cell death protein 1 (PD-1) and a use thereof, and more specifically, to: an asymmetric siRNA including an antisense strand including a sequence complementary to mRNA encoding PD-1, and a sense strand forming complementary bonds with the antisense strand; a pharmaceutical composition for preventing or treating cancer, including the asymmetric siRNA; an immune cell, in which expression of PD-1 is inhibited, obtained by treatment with the siRNA; and an immune cell therapeutic agent for treating cancer, including the immune cell. The present patent application claims priority to Korean Patent Application No. 10-2019-0058805 filed with the Korean Intellectual Property Office on May 20, 2019, and the disclosures of the patent application are incorporated herein by reference.

### BACKGROUND ART

Programmed cell Death protein 1 (PD-1) is one of the receptors expressed on the cell surface and inhibits the activity of immune cells. When T lymphocytes, which play a major role in the occurrence of an acquired immune response, are activated, the expression of PD-1 is induced. Its interaction with its ligands, PD-L1 and/or PD-L2, results in inhibition of the signaling system initiated by T cell receptors (TCRs) that receive stimulatory signals from the antigen. This process is done by inactivating Zap70, the effector molecule of TCRs, by PD-1 using SHP-2, which is a phosphatase.

This interaction of PD-1/PD-L suppresses the autoimmune reaction from occurring in the normal state, and in the case of disease conditions such as viral infection, immune cells are excessively activated to prevent unintended damage to cells in normal tissues. Therefore, PD-1/PD-L regulates the immune checkpoint.

PD-1 is usually expressed in most immune cells such as activated T cells, B cells, and macrophages, and PD-L1/L2 is expressed on the surface of antigen-presenting cells or stromal cells.

In the case of cancer cells, these immune suppression mechanisms are used to escape from the monitoring of the immune system and create a good environment for cancer cells to grow and penetrate. A typical method is to overexpress PD-L1 in cancer cells, thereby causing T cells to reach an anergy state using the PD-1/PD-L1 interaction. T cells in an anergic state do not proliferate, no longer secrete cytokines, and cannot mediate cytotoxic reactions. By presenting such PD-L1, cancer cells grow and infiltrate tissues avoiding the immune function of immune cells, which plays a major role in the immune system, resulting in a result of inhibiting the effects of cell therapy.

For example, in one study, tumor cells expressing PD-L1 were less susceptible to cytolytic responses induced by cytotoxic T cells, and tumor formation and invasiveness increased in an *in vivo* model in mice. (Iwai, Yoshiko, et al. "Involvement of PD-L1 on tumor cells in the escape from host immune system and tumor immunotherapy by PD-L1 blockade." Proceedings of the National Academy of Sciences 99.19 (2002): 12293-12297). In addition, it has also been confirmed that the activity of cytotoxic T cells is significantly reduced only by PD-L1 expression (Juneja, Vikram R., et al. "PD-L1 on tumor cells is sufficient for immune evasion in immunogenic tumors and inhibits CD8 T cell cytotoxicity." Journal of Experimental Medicine (2017): jem-20160801).

Research has been conducted to inhibit the PD-1/PD-L interaction of these cancer cells, and studies have been reported to treat cancer in a way that the action of PD-1/PD-L1 is competitively or non-competitively inhibited by antibodies that complementarily bind to PD-1 or PD-L. (Rizvi NA, Hellmann MD, Snyder A, Kvistborg P, Makarov V, Havel JJ, et al. (April 2015). "Cancer immunology. Mutational landscape determines sensitivity to PD-1 blockade in non-small cell lung cancer". Science. 348 (6230): 124-128).

However, as side effects of these PD-1/PD-L1 inhibitory antibody treatments, dermatologic toxicities, digestive tract diseases, endocrine toxicities, hepatic toxicities, pneumonia, neurologic syndromes, ocular toxicities, renal toxicities, and pancreatic toxicities have been reported. (J. Naidoo, et al. "Toxicities of the Anti-PD-1 and Anti-PD-L1 Immune Checkpoint Antibodies" Annals of Oncology, mdv383. doi:10.1093/annonc/mdv383).

The treatment of diseases using RNA interference may treat diseases more safely because siRNA that controls gene expression at a translational level targets mRNA. Small interfering RNA (siRNA) is composed of a sense strand having the same sequence as the target mRNA and an antisense strand having a sequence complementary to the target mRNA. Conventional siRNA has a short duplex of 19bp to 21 bp and two nucleotides protruding from both strands 3'. siRNA enters the cell, attaches to the target mRNA, degrades the target mRNA, and suppresses the expression of the target gene. Since it is possible to target all mRNAs by changing the sequence of oligos, it is possible to suppress the expression of proteins with complex structures, thus allowing treatment of diseases such as cancer, viral infection, and genetic diseases that are currently difficult to treat. However, siRNA introduced into cells may cause side effects such as inducing an immune response and suppressing non-target genes. Among the side effects, a delivery system that introduces siRNA into cells is the biggest problem in the development of therapeutic agents using siRNA.

siRNA is negatively charged due to the phosphate backbone, but siRNA has a repulsive force against a negatively charged cell membrane, and thus, a delivery system is required to introduce siRNA into cells. As an example of the delivery system, a method of introducing into cells by offsetting negative charges by wrapping siRNA with liposomes or polymers having positive charges has been widely used. However, a carrier having a positive charge may cause various side effects, such as attaching to a cell membrane having a negative charge, exhibiting unwanted toxicity, or forming an unwanted complex through interaction with various types of proteins in the cell. Also, since siRNA is rapidly degraded by nucleases in the blood, the amount of siRNA reaching the target cell may not be sufficient to significantly reduce the expression of the target gene. Therefore, there is a need for a method in which siRNA may be safely and effectively delivered into target cells.

In this regard, the present inventors have developed a technology related to an asymmetric shorter duplex siRNA (asiRNA) structure (WO 2009/078685). asiRNA is an asymmetric RNAi-derived structure having a short length of a double helix compared to the 19+2 structure. The use of asiRNA is a technology that overcomes problems such as off-target effects of siRNA, saturation of RNAi mechanisms, and immune response by TLR3, and thus, the technology may be a promising platform for the development of RNAi drugs. In addition, chemical and structural modifications are added to allow self-delivery into cells without a separate delivery system, thereby overcoming problems with existing siRNA delivery systems and enabling the development of effective next-generation therapeutic agents.

Accordingly, the present inventors predicted that by inhibiting the expression of PD-1 through RNA interference, the mechanism of immune cell suppression due to overexpression of PD-L1 in cancer cells may be prevented, and thus, the present inventors have been working to invent a nucleic acid molecule capable of inducing RNA interference and having intracellular permeability for effective delivery of the nucleic acid molecule targeting PD-1 into cells. As a result, an asymmetric shorter duplex small interfering RNA (asiRNA) was developed that inhibits the expression of target genes by inducing RNA interference and reduces off-target effects at the same time. In addition, through various chemical modifications, it was confirmed that the asymmetric double-stranded nucleic acid molecule has excellent target gene suppression efficiency without a carrier *in vitro,* and the present invention was completed.

The information described in the background is only for improving understanding of the background of the present invention, and thus may not include information forming the prior art known to those of ordinary skill in the art to which the present invention belongs.

### DESCRIPTION OF EMBODIMENTS

### TECHNICAL PROBLEM

The purpose of the present invention is to provide an asymmetric shorter duplex siRNA (asiRNA) that may specifically inhibit expression of programmed cell death protein 1 (PD-1).

Another purpose of the present invention is to provide a pharmaceutical composition for preventing or treating cancer including the asymmetric siRNA.

Another purpose of the present invention is to provide an immune cell in which expression of PD-1 is suppressed by the asymmetric siRNA.

Another purpose of the present invention is to provide an immune cell therapeutic agent for treating cancer including the immune cells.

### SOLUTION TO PROBLEM

To achieve the foregoing purposes, the present invention provides an asymmetric siRNA including: an antisense strand including a sequence complementary to mRNA encoding programmed cell death protein 1 (PD-1); a sense strand forming complementary bonds with the antisense strand, wherein a 5' end of the antisense strand and a 3' end of the sense strand may form a blunt end.

The present invention also provides a pharmaceutical composition for preventing or treating cancer including the asymmetric siRNA.

The present invention also provides an immune cell in which expression of PD-1 is suppressed by the asymmetric siRNA.

The present invention also provides an immune cell therapeutic agent for treating cancer including the immune cells.

### ADVANTAGEOUS EFFECTS OF DISCLOSURE

According to the present invention, an asymmetric siRNA, which may cause RNA interference effect by targeting mRNA encoding PD-1 of immune cells closely related to immunosuppression and growth and metastasis of cancer through the immunosuppression by anergy of immune cells through the interaction of PD-1/PD-L, may be screened, the siRNA may be introduced into cells without help of a carrier and have a resistance to nucleases by chemically modification to remove cell toxicity due to the carrier, and gene expression *in vivo* may be more efficiently suppressed, and thus, the asymmetric siRNA may be usefully used as a preventive or therapeutic agent for cancer.

### BRIEF DESCRIPTION OF DRAWINGS

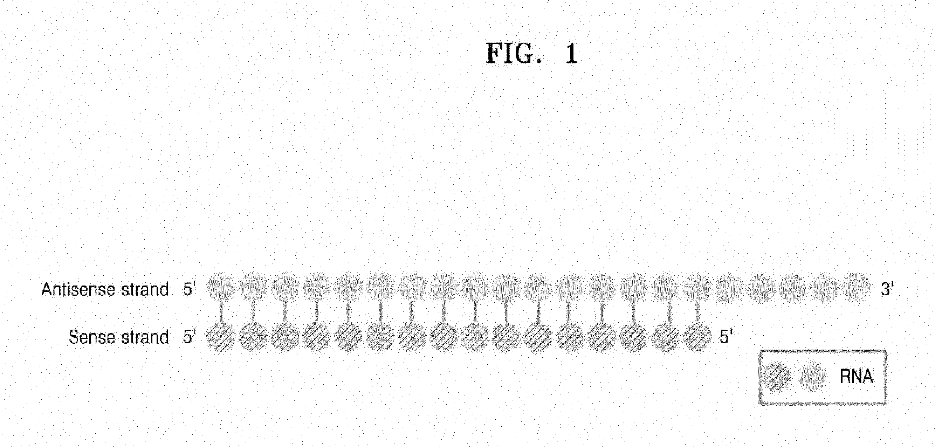

FIG. 1 is a schematic view of a structure of an asymmetric siRNA targeting PD-1, consisting of a sense strand (16 mer) and an antisense strand (21mer).
FIG. 2 is a graph that shows PD-1 mRNA levels (n=3) obtained by transfection of an asymmetric siRNA at a concentration of 10 nM and shows a degree of expression in percent (%) using tubulin as a housekeeping gene based on an untreated sample (control: NT). siOCT4 was used as a negative control.
FIG. 3 is a graph that shows PD-1 mRNA levels (n=3) obtained by transfection of an asymmetric siRNA at a concentration of 1 nM and shows a degree of expression in % using tubulin as a housekeeping gene based on an untreated sample (control: NT). siOCT4 was used as a negative control.
FIG. 4 is a graph that shows PD-1 protein levels (n=4) obtained by ELISA after transfection of asymmetric siRNAs (asiPD-1-93, asiPD-1-95, and asiPD-1-89) effective in suppressing expression of PD-1.
FIG. 5 shows results of western blotting for PD-1 protein levels (n=3), showing inhibitory effects of 12 types of cp-asiRNA shown in Table 3 on PD-1 expression.
FIG. 6 shows results of western blotting for PD-1 protein levels (n=3), showing inhibitory effects of 11 types of cp-asiRNA shown in Table 4 on PD-1 expression.
FIG. 7 shows results of western blotting for PD-1 protein levels (n=3), showing results of a duration test (2 days to 5 days) on 2 types of cp-asiRNAs (cp-asiPD-1-22 and cp-asiPD-1-23) showing high efficiency among 11 types of cp-asiRNAs shown in Table 4.
FIG. 8 is a graph that shows measurement values of luciferase luminescence (n=3) in an average relative light unit 4 days (96 hours) after treating cp-asiRNA with a concentration of 0.5 µM, 1 µM, or 3 µM, showing an increase in T cell activation by inhibiting PD-1 expression of cp-asiPD-1-22 and cp-asiPD-1-23 confirmed in FIG. 7.
FIG. 9 is a graph that shows measurement values of luciferase luminescence (n=3) in an average relative light unit 3 days after treating cp-asiRNA with a concentration of 1 µM under the same condition as FIG. 8, showing an increase in T cell activation by inhibiting PD-1 expression of cp-asiPD-1-22 and cp-asiPD-1-23.
FIG. 10 shows degrees of expression of PD-1 mRNA in % based on an untreated sample (control: NT), showing inhibitory effects on PD-1 expression of 8 types of asiRNA shown in Table 5.
FIG. 11 shows results of western blotting for PD-1 protein levels, showing inhibitory effects of 6 types (8-039, 8-046, 8-048, 8-053, 8-068, and 8-070) showing high efficiency among 8 types of asiRNA shown in Table 5 on PD-1 expression.
FIG. 12 shows degrees of expression of PD-1 mRNA in % based on an untreated sample (control: NT), showing inhibitory effects on PD-1 expression of 12 types of cp-asiRNA shown in Table 6.
FIG. 13 shows degrees of expression of PD-1 mRNA in % based on an untreated sample (control: NT), showing inhibitory effects on PD-1 expression of 4 types (cp-8-068-3, 8-068-13, 8-039-8, and 8-039-13) showing high efficiency among 12 types of cp-asiRNA shown in Table 6.
FIG. 14 shows results of western blotting for PD-1 protein levels, showing inhibitory effects of 4 types (8-068-3, 8-068-13, 8-039-8, and 8-039-13) showing high efficiency among 12 types of cp-asiRNA shown in Table 6 on PD-1 expression.
FIG. 15 shows degrees of expression of PD-1 mRNA in % based on an untreated sample (control: NT), showing inhibitory effects on PD-1 expression in T cells of 4 types of cp-asiRNA (8-068-3, 8-068-13, 8-039-8, and 8-039-13).
FIG. 16 shows relative values of measurement values of luciferase luminescence based on an untreated sample (control: NT) after treating cp-asiRNA with concentrations of 1 µM and 2 µM, showing an increase in T cell activation by inhibiting PD-1 expression of cp-asiRNAs 8-068-3, 8-068-13, 8-039-8, and 8-039-13 confirmed in FIG. 15.

### MODE OF DISCLOSURE

Unless otherwise defined, all technical and scientific terms used in this specification have the same meaning as commonly understood by an expert skilled in the art to which the present invention belongs. In general, the nomenclature used in this specification is well known and commonly used in the art.

Definitions of main terms used in the detailed description of the present invention are as follows.

The term "RNA interference (RNAi)" refers to a mechanism that inhibits expression of a target gene by inducing degradation of mRNA of the target gene by introducing a double-stranded RNA (dsRNA) consisting of a strand having a sequence homologous to mRNA of the target gene and a strand having a sequence complementary thereto.

The term "small interfering RNA (siRNA)"refers to a short double-stranded RNA (dsRNA) that mediates sequence-specific efficient gene silencing.

The term "antisense strand" is a polynucleotide that is substantially or 100 % complementary to a target nucleic acid of interest, such as messenger RNA (mRNA), non-mRNA RNA sequences (e.g., microRNA, piwiRNA, tRNA, rRNA, and hnRNA), or coding or non-coding DNA sequences, which may be complementary in whole or in part.

The term "sense strand" is a polynucleotide that has an identical nucleic acid sequence to a target nucleic acid, such as messenger RNA(mRNA), non-mRNA RNA sequences (e.g., microRNA, piwiRNA, tRNA, rRNA, and hnRNA) or coding or non-coding DNA sequences, which may be identical in whole or in part.

The term "gene" should be considered in the broadest sense and may encode a structural or regulatory protein. In this case, the regulatory protein includes a transcription factor, a heat shock protein, or a protein involved in DNA/RNA replication, transcription, and/or translation.

The term "PD-1" is an abbreviation of programmed cell death protein 1 and belongs to Type I transmembrane protein of a member of the CD28 family, and the human PD-1 gene exists on 2q37.35 chromosome and contains about 55 kD of transmembrane protein. PD-1 is widely expressed on surfaces of activated T cells, B cells, mononuclear cells and dendritic cells, the structure of PD-1 has 30 % homology to CTLA-4, and two tyrosine residues are present in the intracellular region, which may respectively participate in forming of one immunoreceptor tyrosine-based inhibitory motif (ITIM) at the N-terminus and one immunoreceptor tyrosin-based switch motif (ITSM) at the C-terminus. The extracellular region is consisting of one IgV-like structural region, contains a number of glycosylation sites, and is severely glycosylated, and the structural region is bound to a ligand, thereby inhibiting T cell activation. PD-L1 is expressed in excess in many cancer tissues, including non-small cell lung cancer (NSCLC), melanoma, breast cancer, glioma, lymphoma, leukemia, and various urinary system cancers, digestive tract cancers, reproductive system tumors, and the like. The high expression of PD-L1 regulates expression of proteins at a cell cycle detection point and proteins related to cell proliferation through inhibition of RAS and PI3K/AKT signaling pathways, resulting in inhibition of T cell proliferation. It is known that activation of the PD-1/PD-L1 signaling pathway *in vitro* and in mouse models induces specific CTL apoptosis and promotes immune escape of tumor cells by lowering sensitivity of CTL apoptosis effects.

The purpose of the present invention may be broadly expressed in two aspects. The first purpose is to design an asymmetric siRNA targeting an mRNA encoding PD-1 and select an asymmetric siRNA that most efficiently inhibits expression of PD-1 through screening. The second purpose is to introduce a chemical modification to deliver siRNA into cells without any other carrier and increase resistance to nucleases. An siRNA must be small or hydrophobic to pass through cell membrane consisting of phospholipids. However, siRNA is negatively charged by a phosphate backbone, and thus it is difficult for the siRNA to penetrate the cell membrane. In addition, it is necessary to increase resistance to nuclease s for siRNA to have a long lifetime in serum such that the amount reaching the target may be sufficient to generate effective RNAi. Therefore, by introducing chemical modifications, the siRNA delivery problem was overcome.

In one embodiment of the present invention, an asiRNA targeting PD-1 mRNA was designed, and an asiRNA having the best knockdown efficiency was identified and selected. In addition, asiRNA may be modified by introducing chemical modification to have cell-penetrating ability and resistance to nucleases without aid of a carrier, and thus, cp-asiRNA that may efficiently inhibit PD-1 expression was chosen. 4 modifications may be introduced to the chosen siRNA for asiRNA to have cell-penetrating ability and resistance to nucleases. First, cholesterol may be added to 3' end of a sense strand to allow siRNA to penetrate cell membrane. Second, by substituting a phosphate backbone near 5' or 3' end of the sense strand and the antisense strand with phosphorothioate, the siRNA may have resistance to exonucleases, and absorption into cells and bioavailability of siRNA *in vivo* may be available. Third, by modifying 2'-OH group of sugar with 2'-O-methyl, resistance to nucleases may be obtained, and siRNA immunogenicity may be lowered, thus reducing off-target effects. Fourth, 2' of sugar may be modified with fluorine to grant stability to double stranded duplex, thus increasing stability in serum and enabling efficient silencing *in vitro* and *in vivo.* By applying the above modification to siRNA, siRNA may have cell-penetrating ability, and siRNA may stay in serum for a longer time and allow more efficient gene suppression as sufficient amount of siRNA is delivered to target cells.

Accordingly, in an aspect of the present invention, an asymmetric siRNA may include: an antisense strand including a sequence complementary to mRNA encoding programmed cell death protein 1 (PD-1); a sense strand forming complementary bonds with the antisense strand, wherein a 5' end of the antisense strand and a 3' end of the sense strand may form a blunt end.

The siRNA in the present invention may include all substances having a general RNA interference (RNAi) action. Therefore, siRNA as used herein is not necessarily limited to synthetic siRNA, and may be applied to siRNA or shRNA expressed in cells using an expression vector or the like. RNAi is an intracellular gene regulation method first discovered in *Caenorhabditis elegans* by Fire research group in 1998. The action mechanism is that an antisense strand of an RNA double strands injected into a cell complementarily binds to an mRNA of a target gene to induce target gene degradation. Among them, synthetic RNA interference (siRNA) is one of the methods to suppress gene expression *"in vitro".* siRNA having 19 bp to 21 bp is theoretically a technology that may be developed as a therapeutic agent for various gene-related diseases such as cancer, rare diseases, fibrosis, and viral infections as siRNA may selectively suppress most of genes. In mid-2002, *in vivo* treatment with siRNA was first attempted in mammals, and there many attempts have been performed on applied research since then. In particular, in 2018, Onpattro, the first siRNA-based RNAi treatment for multiple neuropathies of hereditary transthyretin-mediated (hATTR) amyloidosis by Alnylam Pharmaceuticals, was approved by the U.S. Food and Drug Administration (FDA) and the European Medicines Agency (EMA). Onpattro was previously designated as an innovative treatment and an orphan drug. Many other global pharmaceutical companies are investing more than billions of dollars on RNAi therapeutic projects, including siRNA, for various intractable diseases. However, existing RNAi therapeutics still have barriers such as 1) lack of an effective delivery system 2) off-target effect 3) immune response induction 4) saturation of intracellular RNAi pathway. Thus, there is a need to overcome these barriers. Due to these problems, although siRNA is an effective method to directly control expression of a target gene, it is difficult to develop a therapeutic agent.

To solve this, the present invention proposes an asymmetric siRNA including an antisense strand and a sense strand complementary to the antisense strand, and the siRNA according to the present invention may not cause problems such as off-target effect and saturation of RNAi mechanisms. Therefore, it is possible to stably suppress expression of the PD-1 target gene while maintaining high delivery efficiency to a desired degree.

In the present invention, the sense strand of the siRNA may have a length of 15 nt to 17 nt, and the antisense strand may have a length of 16 nt or more. Although not limited thereto, the antisense strand may have a length of 16 nt to 31 nt or preferably a length of 19 nt to 25 nt. More preferably, the sense strand may have a length of 16 nt, and the antisense strand complementary thereto may have a length of 19 nt, 21 nt, or 25 nt, but is not limited thereto. 3' end of the sense strand and 5' end of the antisense strand may form a blunt end. 3' end of the antisense strand may include, for example, overhang of 1 nt to 16 nt.

In some embodiments, 36 asiRNAs were designed to suppress PD-1 expression, and inhibition in HeLa-PD-1 cell expressing PD-1 in mRNA level and protein level were identified.

In the present invention, the sense strand may be selected from the group consisting of SEQ ID NOs:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 77, 79, 81, 83, 85, 87, 89, and 91. Preferably, an siRNA including the sense strand selected from the group consisting of SEQ ID NOs:43, 47, 49, 77, and 89 and an antisense strand to complementary the sense strand was found to be most effective in inhibiting PD-1 expression.

In the present invention, the antisense strand may be selected from the group consisting of SEQ ID NOs:2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 78, 80, 82, 84, 86, 88, 90, and 92. Preferably, the antisense strand may be selected from the group consisting of SEQ ID NOs:44, 48, 50, 78, and 80.

More preferably, the sense strand of the siRNA of the present invention may be SEQ ID NO:43, 47, 49, 77, or 89, and the antisense strand of the siRNA may be SEQ ID NO:44, 48, 50, 78, or 90.

In the present invention, the sense strand or the antisense strand of the siRNA may include at least one chemical modification.

General siRNA may not pass through cell membrane due to high molecular weight and high negative charge due to the phosphate backbone structure, and it is difficult to deliver a sufficient amount for RNAi induction to the actual target site because general siRNA is rapidly degraded and removed from the blood. Currently, in the case of *in vitro* delivery, many high-efficiency delivery methods using cationic lipids and cationic polymers have been developed, but in the case of *in vivo,* it is difficult to deliver siRNA with as high efficiency as *in vitro,* and there is a problem in that siRNA delivery efficiency decreases due to interaction with various proteins present *in vivo.*

Accordingly, the present inventors have developed an asiRNA construct (cp-asiRNA) having an effective and self-transmitting ability capable of intracellular delivery without a separate carrier by introducing chemical modifications to an asymmetric siRNA structure.

In the present invention, chemical modification in the sense strand or the antisense strand may include at least one selected from the group consisting of: -OH group at 2' carbon position in a sugar structure of a nucleotide substituted with methyl (-CH₃), methoxy (-OCH₃), -NH₂, -F(fluorine), -O-2-methoxyethyl -O-propyl, -O-2-methylthioethyl, -O-3-aminopropyl, -O-3-dimethylaminopropyl; oxygen in a sugar structure of a nucleotide substituted with sulfur; a phosphate bond of a nucleotide modified with phosphorothioate, phosphorodithioate, boranophosphate, or methyl phosphonate; substitution with a peptide nucleic acid (PNA), a locked nucleic acid (LNA), or an unlocked nucleic acid (UNA); and a phosphate group, lipophilic compound, or cell-penetrating peptide bond.

In the present invention, the lipophilic compound may be selected from the group consisting of cholesterol, tocopherol, docosahexaenoic acid (DHA), palmitic acid, and long-chain fatty acids having 10 or more carbon atoms. Preferably, the lipophilic compound may be cholesterol, but embodiments are not limited thereto.

In some embodiments, the sense strand or the antisense strand may include at least one modification selected from the group consisting of: -OH group at 2' carbon position in a sugar structure of a nucleotide substituted with methoxy (-OCH₃) or - F(fluorine); at least one nucleotide bond in the sense strand or the antisense strand modified with phosphorothioate; 3' end of the sense strand modified with a lipophilic compound bond; and 5' end of the antisense strand modified with a phosphate group bond.

The sense strand in the siRNA in the present invention may be selected from the group consisting of (a) to (c), and the antisense strand may be selected from the group consisting of (d) to (o).

| | Sequence (5' →3') |
|---|---|
| Sense | |
| (a) | mCAmGGmGUmGAmCAmGAmGA·mG·A·/chol/ |
| (b) | mAGmGGmUGmACmAGmAGmAG*mA*A*/chol/ |
| (c) | mCAmUGmAGmCCmCCmAGmCA*mA*C*/chol/ |
| Antisense | |
| (d) | UCUCUCUGUCACCCmU*mG*A*G*C |
| (e) | UCUGUCUGUCACCQmU*mG*mA*mG*mC |
| (f) | UCUCUCUGUCACCCmUmGmAmGmCmUmC*mU*mG*mC*mC |
| (g) | UCUCUCUGUCACCCmUmGmAmGmCmUmC*mU*G*C*C |
| (h) | UCUCUCUGUCACCCmUmGmAniGmCmUC*U*G*C*C |
| (i) | UUCUCUCUGUCACCmC*mU*G*A*G |
| (j) | UUCUCUCUGUCACCmC*mU*mG*mA*mG |
| (k) | UUCUCUCUGUCACCmCmUmGmAmGmCrnU*mC*mU*mG*mC |
| (l) | UUCUCUCUGUCACCmCmUmGmAmGmCmU*mC*U*G*C |
| (m) | UUCUCUCUGUCACCmCmUmGmAmGmCU*C*U*G*G |
| (n) | GUUGCUGGGGCUCAmU*mG*mC*mG*mG |
| (o) | GUUGCUGGGGCUCAmU*mG*C*G*G |

In the table above, * indicates a phosphorothioated bond, m indicates 2'-O-methyl, and /chol/ indicates cholesterol. Specifically, "*" refers to an existing phosphodiester bond substituted with a phosphorothioate bond, "m" refers to 2'-OH substituted with 2'-O-methyl. In the case of "2'-F-", for example, "2'-F-G" refers to existing 2'-OH of guanine (G) substituted with fluorine, and "Chol" refers to a form in which cholesterol is added at 3'-end. 5' end of the antisense strand may further be bonded with 1 to 3 phosphate group(s).

Preferably, the sense strand may be (b) in the table, and the antisense strand may be (i) or (m) in the table.

More preferably, the sense strand may be selected from the group consisting of (a) to (c), and the antisense strand may be selected from the group consisting of (d) to (h):

| | Sequence (5' →3') |
|---|---|
| Sense | |
| (a) | mAGmGGmUGmACmAGmAGmAG*mA*A*/chol/ |
| (b) | mA(2'-F-G)mG(2'-F-G)mU(2'-F-G)mA(2'-F-C)mA(2'-F-G)mA(2'-F-G)mA(2'- F-G)*mA*(2'-F-A)*/chol/ |
| (c) | mAmGmGGmUGAmCAGAGAmG-mA*mA*/chol/ |
| Antisense | |
| (d) | 5' P-UUCUCUCUGUCACCmCmUmGmAmGmCU*C*U*G*C |
| (e) | 5' P-mU•(2'-F-U)•mC(2'-F-U)mC(2'-F-U)mC(2'-F-U)mG(2'-F-U)mC(2'-F-A) mC(2'-F-C)mC(2'-F-U)mG(2'-F-A)mG(2'-F-C)mU•(2'-F-C)•mU•(2'-F-G)•mC |
| (f) | 5' P—mU*(2'—F—U)*(2'—F—C)(2'—F—U)(2'—F—C)(2'—F—U)(2'—F—C)(2'—F—U)G(2'—F—U) (2'—F—C)A(2'—F—C)CCUmGmAmGmCU*C*U*G*C |
| (g) | 5' P-UCUCUCUGUCACCCmUmGmAmGmCmUmC*mU*mG*mC*mC |
| (h) | 5' P-UCUCUCUGUCACCCmU*mG*mA*mG*mC |

In the table above, * indicates a phosphorothioated bond, m indicates 2'-O-methyl, 2'-F- indicates 2'-fluoro, /chol/ indicates cholesterol, and 5'P indicates a 5'-phosphate group.

Most preferably, the sense strand may be (c) in the table, and the antisense strand may be (g) or (h) in the table.

Preferably, the sense strand may be one selected from the group consisting of (A) to (D), antisense strand may be one selected from the group consisting of (E) to (H), and more preferably, the siRNA may be: Sense Strand (A) and Antisense Strand (E); Sense Strand (B) and Antisense Strand (F); Sense Strand (C) and Antisense Strand (G); or Sense Strand (D) and Antisense Strand (H).

| | Sequence (5' →3') |
|---|---|
| Sense | |
| (A) | GGAGmUAmUGmCmCAmCmCA*mU*mU*/chol/ |
| (B) | mG(2'F-G)mA(2'F-G)mU(2'F-A)mU(2'F-G)mC(2'F-C)mA(2'F-C)mC(2'F-A) *mU*(2'F-U)*/chol/ |
| (C) | mCUmAAmACmUGmGUrnACmCG*mC*A*/chol/ |
| (D) | mC(2'-F-U)mA(2'-F-A)mA(2'-F-C)mU(2'-F-G)mG(2'-F-U)mA(2'-F-C)mC (2'-F-G)*mC*(2'-F-A)*/chol/ |
| Antisense | |
| (E) | |
| (F) | |
| (G) | 5'P-mA(2'F-A)mU(2'F-G)mG(2'F-U)mG(2'F-G)mC(2'F-A)mU(2'F-A)mC(2'F-U) mC*(2'F-C)*mG*(2'F-U)*mC 5'P-mA(2'F-A)mU(2'F-G)mG(2'F-U)mG(2'F-G)mC(2'F-A)mU(2'F-A)mC(2'F-U) mC*(2'F-C)*mG*(2'F-U)*mC 5'P-mU(2'-F-G)mC(2'-F-G)mG(2'-F-U)mA(2'-F-C)mC(2'-F-A)mG(2'-F-U) mU(2'-F-U)mA*(2'-F-G)*mC*(2'-F-A)*mC 5'P-mU(2'-F-G)mC(2'-F-G)mG(2'-F-U)mA(2'-F-C)mC(2'-F-A)mG(2'-F-U) mU(2'-F-U)mA*(2'-F-G)*mC*(2'-F-A)*mC |
| (H) | |

In the table above, * indicates a phosphorothioated bond, m indicates 2'-methyl, 2'-F- indicates 2'-fluorine (F), /chol/ indicates cholesterol, and 5'P indicates a 5'-phosphate group.

Another aspect of the present invention provides a pharmaceutical composition for preventing or treating cancer including the siRNA.

In the present invention, the cancer may be selected from multiple myeloma, non-epithelial tumor, non-small cell lung cancer (NSCLC), melanoma, breast cancer, glioma, lymphoma, leukemia, urinary system cancer, digestive tract cancer, reproductive system tumor, refractory classic Hodgkin's lymphoma, prostate cancer, metastatic melanoma, renal cell carcinoma, Hodgkin lymphoma, squamous cell carcinoma of the head and neck, urinary tract epithelial cell carcinoma, refractory B cell precursor acute lymphocytic leukemia, and diffuse large B cell lymphoma, but is not limited thereto, and any cancer known to express PD-1 ligand represented by PD-L1 may be treated with a composition for preventing or treating cancer without any limitation.

The pharmaceutical composition may be prepared by including one or more pharmaceutically acceptable carriers in addition to siRNA as an active ingredient. The pharmaceutically acceptable carrier is compatible with the active ingredient of the present invention and may include saline, sterile water, Ringer's solution, buffered saline, dextrose solution, maltodextrin solution, glycerol, ethanol, or a mixture of two or more of these ingredients. Any other general additives such as antioxidants, buffers, and bacteriostatic agents may be added according to a need. In addition, diluents, dispersants, surfactants, binders, and lubricants may be additionally added to formulate injectable formulations such as aqueous solutions, suspensions, and emulsions. In particular, it is preferable to provide a formulation in a lyophilized form. For the preparation of the lyophilized formulation, a method commonly known in the art may be used, and a stabilizer for lyophilization may be added.

The method of administering the pharmaceutical composition may be determined by one of ordinary skill in the art based on symptoms and severity of the disease of a typical patient. In addition, the pharmaceutical composition may be formulated in various forms such as powders, tablets, capsules, liquids, injections, ointments, syrups, or the like, and the pharmaceutical composition may be provided in unit-dose or multi-dose containers, such as sealed ampoules and bottles.

The pharmaceutical composition of the present invention may be administered orally or parenterally. The route of administration of the composition according to the present invention may be, for example, oral, intravenous, intramuscular, intraarterial, intramedullary, intrathecal, intracardiac, transdermal, subcutaneous, intraperitoneal, intestinal, sublingual, or topical administration, but is not limited thereto. The dosage of the composition according to the present invention may vary depending on the patient's weight, age, sex, health status, diet, administration time, method, excretion rate, or severity of disease and may be determined by one of ordinary skill in the art. In addition, the composition of the present invention may be formulated into a suitable formulation using known techniques for clinical administration.

In another aspect, the present invention relates to a method of preventing or treating cancer including administering the siRNA to an individual. Since the configuration included in impriving or treating according to the present invention is the same as the configuration included in the invention described above, the above description may be identically applied to the improvement or treatment method.

In another aspect, the present invention relates to an immune cell, in which expression of PD-1 is inhibited, obtained by treating the immune cell with the siRNA.

The term "immune cell" as used herein refers to a cell involved in an immune response expressing PD-1. Preferably, an immune cell may be an immune cell used for tumor immunotherapy, but is not limited thereto.

In the present invention, the immune cell may be selected from the group consisting of a natural killer cell (NK cell), a chimeric antigen receptor-modified NK cell (CAR-NK cell), a universal-NK cell, a cytotoxic T lymphocyte (CTL), a tumor-infiltrating T lymphocyte (TIL), a peripheral blood mononuclear cell (PBMC), a T receptor-modified T cell (TCR-T), and a chimeric antigen receptor-modified T cells (CAR-T). Preferably, the immune cell may be CD8+ T cell, but is not limited thereto.

The term "chimeric antigen receptor-T cell (CAR-T cell)" or "CAR-NK cell" as used herein respectively refers to a T cell or NK cell expressing a 'chimeric antigen receptor'.

The term "chimeric antigen receptor (CAR)" as used herein refers to a recombinant polypeptide construct that may provide a target cell and intracellular signal generation to a cell in an immune effector cell. CAR is a molecule that combines antibody-based specificity for a target antigen (e.g., a tumor antigen) with a receptor-activated intracellular domain of T cells or NK cells to produce chimeric proteins that exhibit specific anti-tumor cell immune activity.

The term "chimera" of the present invention refers to a composition of parts of different proteins or DNAs of different origins. The CAR may include at least an extracellular binding domain, a transmembrane domain, and an intracellular signaling domain.

This immune cell-based tumor immunotherapy has been successfully used in clinical practice and has shown unprecedented clinical efficacy. Despite the remarkable effect of immune cell-based tumor immunotherapy, problems have been raised due to various difficulties in the actual tumor treatment process, and one of the most important reasons is the above-described PD-1/PD-L1 inhibitory immune checkpoints. The immune checkpoints significantly reduce effectiveness of the immune cell-based tumor immunotherapy by inhibiting immune activity of immune cells in association with inhibitory signal transduction.

Accordingly, the present inventors developed immune cells with suppressed PD-1 expression obtained by treatment with the siRNA to prevent reduction of the effects of immune cell-based tumor immunotherapy due to inhibitory immune checkpoints with PD-ligand expressed by cancer cells.

In one embodiment of the present invention, when cp-asiRNA was treated on an effector T cell expressing PD-1, a satisfactory activity recovery effect was confirmed even in the presence of PD-L1 expressing cells. In this light, even when the expression of PD-1 is suppressed by siRNA against any of the above-described immune cells such as NK cells, CAR-NK cells, CAR-T, or TCR-T, recovery of the activity of immune cells may be expected.

In another aspect, the present invention relates to an immune cell therapeutic agent for cancer including immune cells in which expression of PD-1 is suppressed.

The term "immune cell therapeutic agent" as used herein refers to a drug used for treating diseases by activating immune response in a body using immune cells such as dendritic cell, natural killer cells, or T cells. The immune cell therapeutic agent is mainly developed as an indication for cancer treatment, and the therapeutic mechanism and efficacy of the immune cell therapeutic agent are differentiated from surgical therapy, therapy using anticancer drugs, or radiation therapy in the related art used in cancer treatment, because therapeutic effects may be obtained by activating immune function by administering immune cells directly to the patient. Thus, the immune cell therapeutic agent occupies a major part of new bio drugs.

According to characteristics of genes introduced into cells during the manufacturing process and characteristics of immune cells used, the immune cell therapeutic agent may include dendritic immunomodulatory cell therapy, natural killer cells (NK cells), and chimeric antigen receptor-modified NK cells (CAR-NK cells). Universal-NK cells, lymphokine activated killers (LAK), tumor-infiltrating T lymphocytes (TIL), T cell receptor-modified T cells (TCR -T), and chimeric antigen receptor-modified T cells (CAR-T) may be included, but is not limited thereto.

In the present invention, the cancer may include breast cancer, multiple myeloma, non-epithelial tumor, NSCLC, melanoma, glioma, lymphoma, leukemia, urinary system cancer, digestive tract cancer, and reproductive system tumor, refractory classic Hodgkin's lymphoma, prostate cancer, metastatic melanoma, renal cell carcinoma, Hodgkin lymphoma, squamous cell carcinoma of the head and neck, urinary tract epithelial cell carcinoma, refractory B cell precursor acute lymphocytic leukemia, and diffuse large B cell lymphoma, but is not limited thereto, and any cancer known to express PD-1 ligand represented by PD-L1 may be treated with the immune cell therapeutic agent without any limitation.

Hereinafter, the present invention will be described in further detail with reference to Examples. The Examples are for illustrative purposes only, and it will be apparent to one of ordinary skill in the art that the scope of the present invention is not construed as being limited by these examples.

Example 1: Screening of 36 RNAi-induced double-stranded nucleic acid molecules targeting PD-1

### 1.1. Confirmation of PD-1 expression in HeLa-PD-1 cell line

Prior to designing asiRNAs and verifying inhibitory effects thereof on PD-1 mRNA expression, expression of PD-1 in HeLa-PD-1 cell lines was confirmed using PCR and ELISA. HeLa-PD-1 stably overexpresses PD-1 with a cell line selected using hygromycin B (Gold Biotechnology Inc.), which is a selection marker, after transfection of a plasmid encoding mRNA of PD-1 into HeLa (ATCC). This feature overcomes the limitation of PD-1 that transcription is induced only by stimulation, rather than being always expressed, and enables smooth screening.

### 1.2. Selection of target PD-1 gene and design and production of 36 asiRNAs

In order to secure a double-stranded nucleic acid molecule that induces high-efficiency RNAi interference targeting PD-1, asiRNA was designed after selecting a target sequence for the PD-1 gene. asiRNA structure has different secondary structure, GC contents (%), 5' end stability difference, or the like, as compared with commonly known siRNA. Thus, when designing a base sequence of asiRNA using a general siRNA design program, it may be difficult to design an optimized asiRNA. Therefore, designing of the candidate asiRNA of the present invention was carried out in the following method.

PD-1 gene information was obtained through NCBI database search (mRNA accession number: NM_005018.2). In consideration of animal experiments, base sequences showing 100 % homology based on a base sequence having a common target with a mouse was first selected, and then base sequences allowing mismatch in the antisense strand except for the seed region (base sequence No. 2 to 8 based on 5') were further selected. Then, 36 asiRNAs were designed and synthesized in 10-nanomole-scale by OliX Pharmaceuticals, Inc.(Korea). The 36 designed asiRNAs each had a base sequence as shown in Table 1. Each of the asiRNAs was diluted to an appropriate concentration in 5x siRNA duplex (available from Bioneer) and annealed through an incubation process at 95 °C for 5 minutes and 37 °C for 1 hour. After performing 12 % polyacrylamide gel electrophoresis (PAGE) thereon, QC through a ChemiDoc UV transilluminator (available from Biorad) was carried out. The 36 screened asiRNAs base sequences are shown in Table 1.

**[Table 1]**

| asiRNA name | Sequence(5' →3') | | SEQ ID NO. |
|---|---|---|---|
| asiPD-1-4 | sense | CUGUGGGGCCAUCUCC | 1 |
| | antisense | GGAGAUGGCCCCACAGAGGUA | 2 |
| asiPD-1-5 | sense | UCUGUGGGGCCAUCUC | 3 |
| | antisense | GAGAUGGCCCCACAGAGGUAG | 4 |
| asiPD-1-11 | sense | CCUUCACCUGCAGCUU | 5 |
| | antisense | AAGCUGCAGGUGAAGGUGGCG | 6 |
| asiPD-1-13 | sense | ACCUUCACCUGCAGCU | 7 |
| | antisense | AGCUGCAGGUGAAGGUGGCGU | 8 |
| asiPD-1-15 | sense | CACCUUCACCUGCAGC | 9 |
| | antisense | GCUGCAGGUGAAGGUGGCGUU | 10 |
| asiPD-1-17 | sense | CUCUGUGGGGCCAUCU | 11 |
| | antisense | AGAUGGCCCCACAGAGGUAGG | 12 |
| asiPD-1-19 | sense | CCUCUGUGGGGCCAUC | 13 |
| | antisense | GAUGGCCCCACAGAGGUAGGU | 14 |
| asiPD-1-21 | sense | ACCUCUGUGGGGCCAU | 15 |
| | antisense | AUGGCCCCACAGAGGUAGGUG | 16 |
| asiPD-1-31 | sense | CCACCUUCACCUGCAG | 17 |
| | antisense | CUGCAGGUGAAGGUGGCGUUG | 18 |
| asiPD-1-33 | sense | GCCCCAGCAACCAGAC | 19 |
| | antisense | GUCUGGUUGCUGGGGCUCAUG | 20 |
| asiPD-1-35 | sense | AGCCCCAGCAACCAGA | 21 |
| | antisense | UCUGGUUGCUGGGGCUCAUGC | 22 |
| asiPD-1-51 | sense | GGCACCUACCUCUGUG | 23 |
| | antisense | CACAGAGGUAGGUGCCGCUGU | 24 |
| asiPD-1-53 | sense | CGGCACCUACCUCUGU | 25 |
| | antisense | ACAGAGGUAGGUGCCGCUGUC | 26 |
| asiPD-1-55 | sense | GCGGCACCUACCUCUG | 27 |
| | antisense | CAGAGGUAGGUGCCGCUGUCA | 28 |
| asiPD-1-57 | sense | AGCGGCACCUACCUCU | 29 |
| | antisense | AGAGGUAGGUGCCGCUGUCAU | 30 |
| asiPD-1-59 | sense | CGUGACUUCCACAUGA | 31 |
| | antisense | UCAUGUGGAAGUCACGCCCGU | 32 |
| asiPD-1-65 | sense | GGGCGUGACUUCCACA | 33 |
| | antisense | UGUGGAAGUCACGCCCGUUGG | 34 |
| asiPD-1-77 | sense | ACUAUGGGGAGCUGGA | 35 |
| | antisense | UCCAGCUCCCCAUAGUCCACA | 36 |
| asiPD-1-79 | sense | GACACUGCUCUUGGCC | 37 |
| | antisense | GGCCAAGAGCAGUGUCCAUCC | 38 |
| asiPD-1-85 | sense | GCCACCUUCACCUGCA | 39 |
| | antisense | UGCAGGUGAAGGUGGCGUUGU | 40 |
| asiPD-1-87 | sense | CGCCACCUUCACCUGC | 41 |
| | antisense | GCAGGUGAAGGUGGCGUUGUC | 42 |
| asiPD-1-89 | sense | CAUGAGCCCCAGCAAC | 43 |
| | antisense | GUUGCUGGGGCUCAUGCGGUA | 44 |
| asiPD-1-91 | sense | GCAUGAGCCCCAGCAA | 45 |
| | antisense | UUGCUGGGGCUCAUGCGGUAC | 46 |
| asiPD-1-93 | sense | AGGGUGACAGAGAGAA | 47 |
| | antisense | UUCUCUCUGUCACCCUGAGCU | 48 |
| asiPD-1-95 | sense | CAGGGUGACAGAGAGA | 49 |
| | antisense | UCUCUCUGUCACCCUGAGCUC | 50 |
| asiPD-1-97 | sense | UCAGGGUGACAGAGAG | 51 |
| | antisense | CUCUCUGUCACCCUGAGCUCU | 52 |
| asiPD-1-99 | sense | GACUAUGGGGAGCUGG | 53 |
| | antisense | CCAGCUCCCCAUAGUCCACAG | 54 |
| asiPD-1-101 | sense | AGUGGCGAGAGAAGAC | 55 |
| | antisense | GUCUUCUCUCGCCACUGGAAA | 56 |
| asiPD-1-103 | sense | CAGUGGCGAGAGAAGA | 57 |
| | antisense | UCUUCUCUCGCCACUGGAAAU | 58 |
| asiPD-1-105 | sense | CCAGUGGCGAGAGAAG | 59 |
| | antisense | CUUCUCUCGCCACUGGAAAUC | 60 |
| asiPD-1-107 | sense | UCCAGUGGCGAGAGAA | 61 |
| | antisense | UUCUCUCGCCACUGGAAAUCC | 62 |
| asiPD-1-109 | sense | UCUGCAGACCCUCCAC | 63 |
| | antisense | GUGGAGGGUCUGCAGAACACU | 64 |
| asiPD-1-116 | sense | ACGCCACCUUCACCUG | 65 |
| | antisense | CAGGUGAAGGUGGCGUUGUCC | 66 |
| asiPD-1-118 | sense | AACGCCACCUUCACCU | 67 |
| | antisense | AGGUGAAGGUGGCGUUGUCCC | 68 |
| asiPD-1-138 | sense | UUCUGCAGACCCUCCA | 69 |
| | antisense | UGGAGGGUCUGCAGAACACUG | 70 |
| asiPD-1-134 | sense | GGACUAUGGGGAGCUG | 71 |
| | antisense | CAGCUCCCCAUAGUCCACAGA | 72 |

### 1.3. Confirmation of PD-1 mRNA expression inhibitory effects of 36 asiRNAs

To identify gene inhibitory efficiency at an mRNA level, transfection with 36 selected asiRNAs at concentrations of 10 nM and 1 nM into HeLa-PD-1 cell lines were performed, and then qRT-PCR was performed thereon to measure the expression level of PD-1 mRNA.

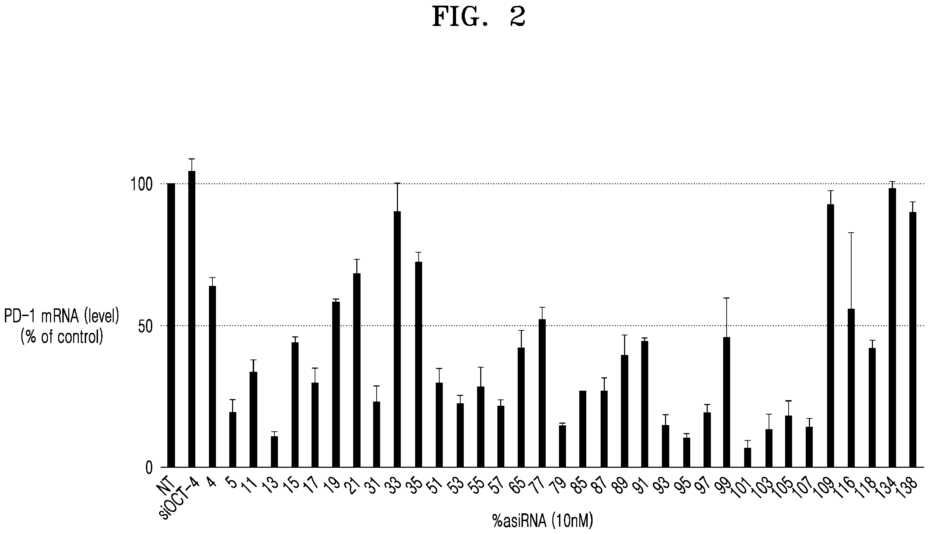

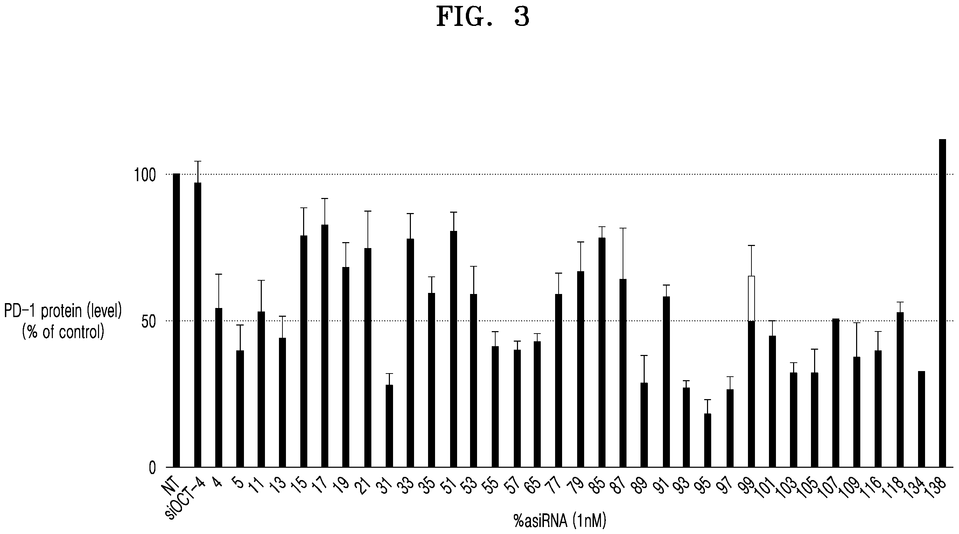

Specifically, HeLa-PD-1 cells were cultured in Dulbecco's modified Eagle's medium (Gibco) to which 10 % fetal bovine serum (Gibco) and 0.2 g/L hygromycin B were added in a 100 mm cell culture dish. HeLa-PD-1 cells cultured 24 hours before transfection were seeded in a 12-well plate. Thereafter, 36 asiRNAs of 1 nM or 10 nM (Table 1) were transfected using RNAimax (Invitrogen) based on the protocol provided by the manufacturer. After 24 hours, the total RNA was extracted using Tri-RNA reagent (FAVORGEN), and 500 ng of RNA was used for cDNA synthesis. cDNA was synthesized by using a High-capacity cDNA reverse transcription kit (Applied Biosystems) according to the protocol provided by the manufacturer. The synthesized cDNA was then subjected to qRT-PCR using the StepOne real-time PCR system (Applied Biosystems). By using the primers of Table 2 and SYBR green PCR master Mix (Applied Biosystems), the amount of PD-1 expression in the asiRNA transfection sample, compared with non-treated sample (NT), was converted into percentage (FIG. 2 (10 nM) and FIG. 3 (1 nM)). Tubulin was used as a housekeeping gene (Table 2).

**[Table 2]**

| Name | | Sequence(5'->3') | SEQ ID NO. |
|---|---|---|---|
| Tubulin | Forward | GACCAAGCGTACCATCCAGT | 73 |
| | Reverse | ACGTTTGGCATACATCAGG | 74 |
| PD-1 | Forward | CCCTGGTGGTTGGTGTCGT | 75 |
| | Reverse | GCCTGGCTCCTATTGTCCCTC | 76 |

The experiment was independently performed three times, and the gene suppression efficiencies of asiRNA against 36 types of sequences targeting PD-1 mRNA are shown in FIG. 2 (10 nM) and FIG. 3 (1 nM). Each value represents the mean±standard deviation value of the independent experiment. As a result of the screening for the total of 36 nucleotide sequences, as shown in FIG. 2, it was confirmed that 25 types inhibited the expression of PD-1 mRNA by 50% or more at a concentration of 10 nM. When the concentration was reduced to 1 nM, it was confirmed that 14 sequences out of a total of 36 nucleotide sequences suppressed the expression of PD-1 mRNA with an efficiency of 50 % or more, as shown in FIG. 3. In particular, as a result of measuring IC50 for asiRNA, which shows more than 80 % or higher gene suppression efficiency, it was confirmed that asiPD-1-93 and asiPD-1-95 had the lowest IC50. Among the sequences, asiPD-1-89 was also selected as a candidate group for chemical modification and self-delivery experiments.

### 1.4. Confirmation of protein expression inhibitory effect through quantification of PD-1 protein level of 3 asiRNAs

From the results of 36 asiRNA screening, the two most efficient asiRNAs (asiPD-1-93 and asiPD-1-95) and additional asiPD-1-89 were selected. After transfection at 1 nM concentration, the PD-1 protein level was identified through a human programmed death 1 (PD-1) ELISA kit (Mybiosoure).

Specifically, HeLa-PD-1 cells were cultured in Dulbecco's modified Eagle's medium (Gibco) to which 10 % fetal bovine serum (Gibco) and 0.2 g/L hygromycin B were added in a 100 mm cell culture dish. HeLa-PD-1 cells cultured 24 hours before transfection were seeded in a 12-well plate. Thereafter, 1 nM of 3 asiRNAs (asiPD-1-89, asiPD-1-93, and asiPD-1-95) were transfected using RNAimax (Invitrogen) based on the protocol provided by the manufacturer. After 24 hours after transfection, the media was replaced with a new medium, and at 48 hours, passage was performed with a 12-well plate again. After that, at 96 hours, harvest was performed to prepare cell lysate using 5X RIPA (self-made) buffer. After quantification through BCA assay (Pierce), the PD-1 protein level was identified by using human programmed death 1 (PD-1) ELISA kit (Mybiosource) according to the protocol provided by the manufacturer.

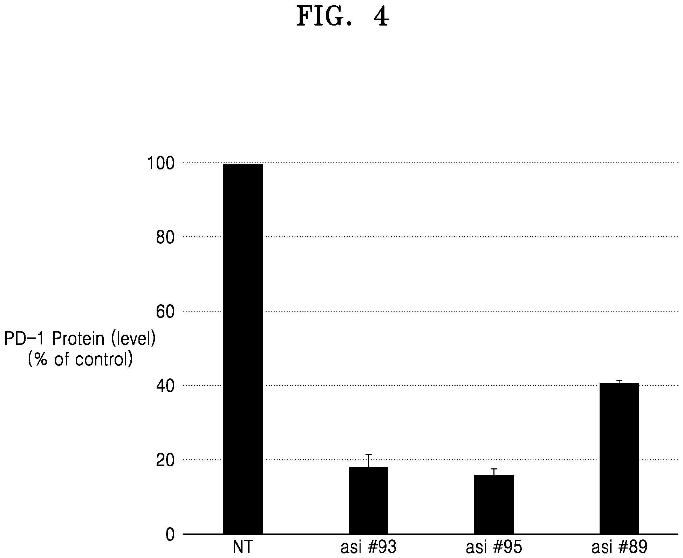

The experiment was independently performed four times, and the PD-1 protein levels (% of control) for the three selected candidate groups are shown in FIG. 4. AsiPD-1-93 and asiPD-1-95, which showed the highest gene suppression efficiency, showed a PD-1 protein level of 20 % or less, as compared with the control (NT), and asiPD-1-89 also showed a PD-1 protein level of about 40 %. Thus, it was confirmed that each candidate group showed a high PD-1 expression inhibitory efficiency.

Example 2: Screening of RNAi-induced asymmetric double-stranded nucleic acid molecules (cp-asiRNA) having cell-penetrating ability targeting PD-1

### 2.1. Design and production of 12 cp-asiRNAs having cell-penetrating ability

Chemical modification, which promote penetration into cells and improve stability of RNA structure, were selected and applied to the three candidate asiRNAs targeting PD-1 (asiPD-1-89, asiPD-1-93, and asiPD-1-95) through two processes. In the first chemical modification, cholesterol was conjugated to the nucleotide at the 3' end of the sense strand, which is 16 nt, and the three phosphate backbones present in the 5' direction were substituted with phosphorothioates. Each hydroxyl group at the 2' position of the ribose of 8 nucleotides of the 16 nucleotides of the sense strand was substituted with an -O-methyl group. In addition, the antisense strand is consisting of 19 nt or 25 nt in length, the hydroxyl group at the 2' position of ribose of 2 nt to 11 nt nucleotides was substituted with -O-methyl group, and the phosphate backbone of 4 nucleotides present in the 3' end direction were substituted with phosphorothioate, thereby designing 12 cp-asiRNAs. The 12 cp-asiRNA nucleotide sequences designed in this manner are shown in Table 3. cp-asiPD-1-1 to cp-asiPD-1-5 were designed based on asiPD-1-95 in Table 1, cp-asiPD-1-6 to cp-asiPD-1-10 were designed based on asiPD-1-93 in Table 1, and cp-asiPD-1-11 to cp-asiPD-1-12 were designed based on asiPD-1-89 in Table 1. Each of the designed cp-asiRNAs was diluted to an appropriate concentration for OptiMEM (Gipco) and annealed through an incubation process at 95 °C for 5 minutes and 37 °C for 1 hour. After performing 12 % polyacrylamide gel electrophoresis (PAGE) thereon, QC through a ChemiDoc UV transilluminator (available from Biorad) was carried out.

**[Table 3]**

| cp-asiRNA name | Sequence(5' →3') | |
|---|---|---|
| cp-asiPD-1-1 | sense | mCAmGGmGUmGAmCAmGAmGA*mG*A*/chol/ |
| | antisense | UCUCUCUGUCACCCmU*mG*A*G*C |
| cp-asiPD-1-2 | sense | mCAmGGmGUmGAmCAmGAmGA*mG*A*/chol/ |
| | antisense | UCUCUCUGUCACCCmU*mG*mA*mG*mC |
| cp-asiPD-1-3 | sense | mCAmGGmGUmGAmCAmGAmGA*mG*A*/chol/ |
| | antisense | UCUCUCUGUCACCCmUmGmAmGmCmUmC*mU*mG*mC*mC |
| cp-asiPD-1-4 | sense | mCAmGGmGUmGAmCAmGAmGA*mG*A*/chol/ |
| | antisense | UCUCUCUGUCACCCmUmGmAmGmCmUmC*mU*G*C*C |
| cp-asiPD-1-5 | sense | mCAmGGmGUmGAmCAmGAmGA*mG*A*/chol/ |
| | antisense | UCUCUCUGUCACCCmUmGmAmGmCmUC*U*G*C*C |
| cp-asiPD-1-6 | sense | mAGmGGmUGmACmAGmAGmAG*mA*A*/chol/ |
| | antisense | UUCUCUCUGUCACCmC*mU*G*A*G |
| cp-asiPD-1-7 | sense | mAGmGGmUGmACmAGmAGmAG*mA*A*/chol/ |
| | antisense | UUCUCUCUGUCACCmC*mU*mG*mA*mG |
| cp-asiPD-1-8 | sense | mAGmGGmUGmACmAGmAGmAG*mA*A*/chol/ |
| | antisense | UUCUCUCUGUCACCmCmUmGmAmGmCmU*mC*mU*mG*mC |
| cp-asiPD-1-9 | sense | mAGmGGmUGmACmAGmAGmAG*mA*A*/chol/ |
| | antisense | UUCUCUCUGUCACCmCmUmGmAmGmCmU*mC*U*G*C |
| cp-asiPD-1-10 | sense | mAGmGGmUGmACmAGmAGmAG*mA*A*/chol/ |
| | antisense | UUCUCUCUGUCACCmCmUmGmAmGmCU*C*U*G*C |
| cp-asiPD-1-11 | sense | mCAmUGmAGmCCmCCmAGmCA*mA*C*/chol/ |
| | antisense | GUUGCUGGGGCUCAmU*mG*mC*mG*mG |
| cp-asiPD-1-12 | sense | mCAmUGmAGmCCmCCmAGmCA*mA*C*/chol/ |
| | antisense | GUUGCUGGGGCUCAmU*mG*C*G*G |
| m:2'-O-CH₃(methyl) modification, * : phosphorothioate linkage, /chol/ : cholesterol | | |

### 2.2. Verification of toxicity and verification of cell penetrating ability and PD-1 expression inhibitory efficiency at protein level for 12 cp-asiRNAs

To confirm the gene inhibitory efficiency at a protein level, cp-asiRNA was treated at a concentration of 1 µM, and then the toxicity caused by cp-asiRNA was identified 24 hours later, and the expression level of PD-1 protein was measured 96 hours after the treatment.

Specifically, first, HeLa-PD-1 cells were cultured in Dulbecco's modified Eagle's medium (Gibco) to which 10 % fetal bovine serum (Gibco) and 0.2 g/L hygromycin B (Gold Biotechnology Inc.) were added in a 100 mm petri dish. HeLa-PD-1 cells cultured 24 hours before the treatment were seeded in a 12-well plate. After seeding, 12 types of cp-asiRNAs were treated at a concentration of 1 µM without a separate carrier.

To confirm toxicity, toxicity caused by cp-asiRNA was confirmed 24 hours after treatment, at 24 hours, the media was changed to new media, and MTT (Sigma-Aldrich) dissolved in Dulbecco's phosphate-buffered saline (DPBS, Gibco) was added thereto such that the final concentration was 5 mg/mL. After incubation at 37° C for 2 hours, all the solutions were removed, DMSO (Sigma-Aldrich) was added, and then incubation was further performed at 37° C for 10 minutes, and absorbance at 570 nm was measured. As a result, it was confirmed that there was no cytotoxicity induced by the prepared cp-asiRNA.

To measure expression of PD-1 protein, the cells were harvested 96 hours after treatment to obtain cell lysate. After quantification using the Thermo Scientific Pierce BCA Protein Assay Kit (Thermo Scientific), the PD-1 protein level was confirmed by Western blotting. Anti-PD1 antibody (Abcam) was used as the primary antibody, and Goat anti-Rabbit igG H&L (HRP) (Abcam) was used as the secondary antibody.

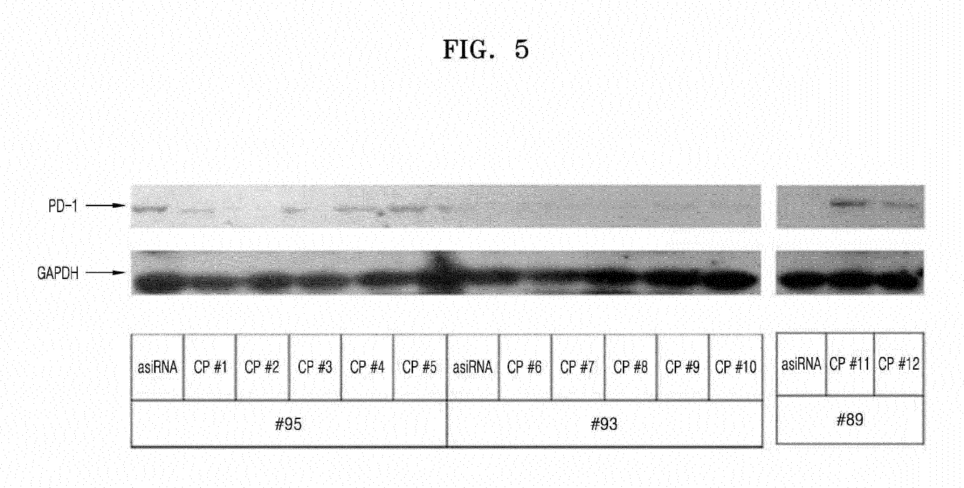

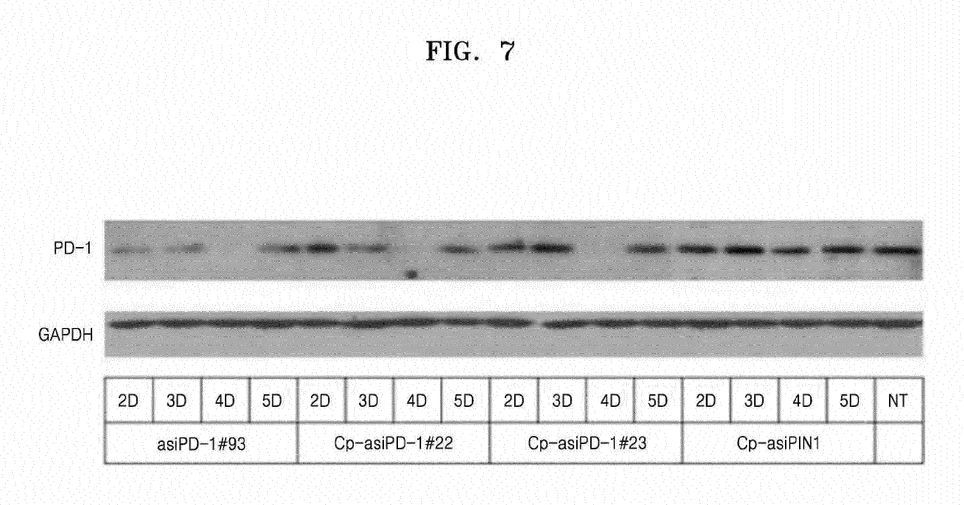

The protein level confirmation was independently performed three times, and FIG. 5 shows the representative result. As a result of the experiment, as shown in FIG. 5, 7 of the 12 cp-asiRNAs were able to exhibit effect of inhibiting gene expression at a protein level. Unlike asiRNA, in the case of cp-asiRNA with chemical modification, it was confirmed that the treatment alone showed a satisfactory effect without a carrier.

### Example 3: Screening of RNAi-induced asymmetric double-stranded nucleic acid molecules (cp-asiRNA) having cell-penetrating ability targeting PD-1

### 3.1. Design and production of 11 cp-asiRNAs of cp-asiPD-1-6 to cp-asiPD-1-10 further chemically modified

cp-asiPD-1-6 to cp-asiPD-1-10 added with chemical modification to asiPD-1-93 of the 7 types, showing the lowest expression level of PD-1 protein among the 12 cp-asiRNAs that were confirmed to be effective in FIG. 5, were further added with chemical modification. Here, the further added modification included conjugation of a phosphate to the nucleotide at the 5' end of the antisense strand or substitution of the hydroxyl groups at the 2' position of the ribose of 10 to 12 nucleotides in both strands with -O-fluoro group or -O-methyl group. The total of 11 cp-asiRNAs were additionally designed and produced, and the nucleotide sequences are as shown in Table 4.

**[Table 4]**

| Cp-asiRNA name | | Sequence(5'→3') |
|---|---|---|
| name cp-asiPD-1-13 | sense | mAGmGGmUGmACmAGmAGmAG*mA*A*/chol/ |
| | antisense | 5'P-UUCUCUCUGUCACCmCmUmGmAmGmCU*C*U*G*C |
| cp-asiPD-1-14 | sense | mAGmGGmUGmACmAGmAGmAG*mA*A*/chol/ |
| | antisense | 5'P-mU*(2'-F-U)*mC(2'-F-U)mC(2'-F-U)mC(2'-F-U)mG(2'-F-U)mC(2'-F-A)mC(2'-F- c)mC(2'-F-U)mG(2'-F-A)mG(2'-F-c)mU*(2'-F-c)*mU*(2'-F-G)*mC |
| cp-asiPD-1-15 | sense | mAGmGGmUGmACmAGmAGmAG*mA*A*/chol/ |
| | antisense | 5'P-mU*(2'-F-U)*(2'-F-c)(2'-F-U)(2'-F-c)(2'-F-U)(2'-F-c)(2'-F-U)G(2'-F-U)(2'-F-c)A(2'- F-c)CCUmGmAmGmCU*C*U*G*C mA(2'-F-G)mG(2'-F-G)mU(2'-F-G)mA(2'-F-c)mA(2'-F-G)mA(2'-F-G)mA(2'-F- G)*mA*(2'-F-A)*/chol/ |
| cp-asiPD-1-16 | sense | |
| | antisense | 5'P-UUCUCUCUGUCACCmCmUmGmAmGmCU*C*U*G*C |
| cp-asiPD-1-17 | sense | mA(2'-F-G)mG(2'-F-G)mU(2'-F-G)mA(2'-F-c)mA(2'-F-G)mA(2'-F-G)mA(2'-F- G)*mA*(2'-F-A)*/chol/ |
| | antisense | 5'P-mU*(2'-F-U)*mC(2'-F-U)mC(2'-F-U)mC(2'-F-U)mG(2'-F-U)mC(2'-F-A)mC(2'-F- c)mC(2'-F-U)mG(2'-F-A)mG(2'-F-c)mU*(2'-F-c)*mU*(2'-F-G)*mC |
| cp-asiPD-1-18 | sense | mA(2'-F-G)mG(2'-F-G)mU(2'-F-G)mA(2'-F-c)mA(2'-F-G)mA(2'-F-G)mA(2'-F- G)*mA*(2'-F-A)*/chol/ |
| | antisense | 5'P-mU*(2'-F-U)*(2'-F-c)(2'-F-U)(2'-F-c)(2'-F-U)(2'-F-c)(2'-F-U)G(2'-F-U)(2'-F-c)A(2'- F-c)CCUmGmAmGmCU*C*U*G*C |
| cp-asiPD-1-19 | sense | mAmGmGGmUGAmCAGAGAmG*mA*mA*/chol/ |
| | antisense | 5'P-UUCUCUCUGUCACCmCmUmGmAmGmCU*C*U*G*C |
| cp-asiPD-1-20 | sense | mAmGmGGmUGAmCAGAGAmG*mA*mA*/chol/ |
| | antisense | 5'P-mU*(2'-F-U)*mC(2'-F-U)mC(2'-F-U)mC(2'-F-U)mG(2'-F-U)mC(2'-F-A)mC(2'-F- c)mC(2'-F-U)mG(2'-F-A)mG(2'-F-c)mU*(2'-F-c)*mU*(2'-F-G)*mC |
| cp-asiPD-1-21 | sense | mAmGmGGmUGAmCAGAGAmG*mA*mA*/chol/ |
| | antisense | 5'P-mU*(2'-F-U)*(2'-F-c)(2'-F-U)(2'-F-c)(2'-F-U)(2'-F-c)(2'-F-U)G(2'-F-U)(2'-F-c)A(2'- F-c)CCUmGmAmGmCU*C*U*G*C |
| cp-asiPD-1-22 | sense | mAmGmGGmUGAmCAGAGAmG*mA*mA*/chol/ |
| | antisense | 5'-P-UCUCUCUGUCACCCmUmGmAmGmCmUmC*mU*mG*mC*mC |
| cp-asiPD-1-23 | sense | mAmGmGGmUGAmCAGAGAmG*mA*mA*/chol/ |
| | antisense | 5'-P-UCUCUCUGUCACCCmU*mG*mA*mG*mC |
| 5'P : 5'-phosphate, m : 2'-O-CH₃ modification, (2'-F-N) : 2'-F modification, * : phosphorothioatelinkane, /chol/ : cholesterol | | |

### 3.2. Verification of toxicity and verification of cell penetrating ability and PD-1 expression inhibitory efficiency at protein level for additionally produced 11 cp-asiRNAs

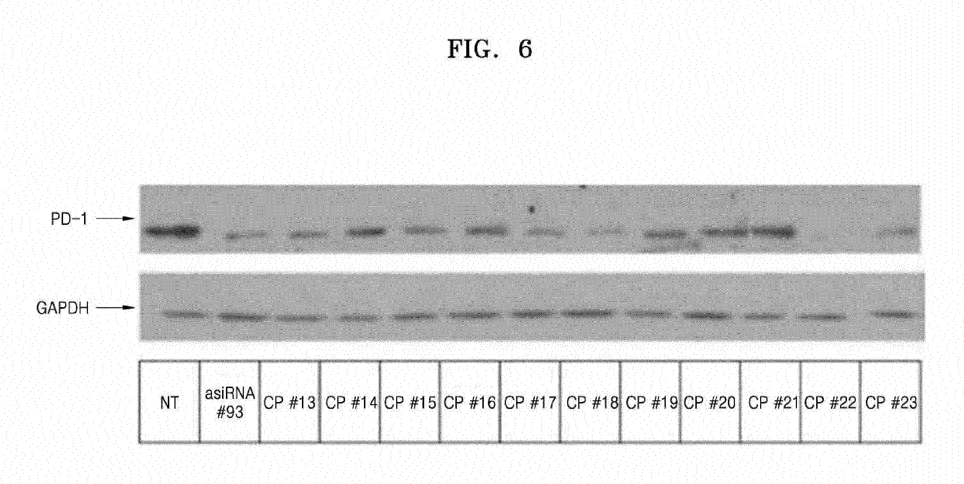

In the same manner as in Example 2.2, the verification of toxicity of 11 additionally produced cp-asiRNAs and verification of the cell penetrating ability and PD-1 expression inhibitory efficiency at the protein level were performed. The experiment was independently conducted three times, and FIG. 6 shows the representative result. As shown in FIG. 6, cp-asiPD-1-22 and cp-asiPD-1-23 were found to be most significant in terms of gene expression inhibitory effects at the protein level.

### Example 4: Duration test for 2 cp-asiRNAs (cp-asiPD-1-22 and cp-asiPD-1-23)

As shown in Example 3, a duration test was performed to confirm the increase in the ability to sustain the effect according to chemical modification to cp-asiPD-1-22 and cp-asiPD-1-23, which showed the best PD-1 expression inhibitory effect. asiPD-1-93 was used as a control, and cp-asiPIN1 was used as a negative control as a cp-asiRNA targeting a gene other than PD-1.

Specifically, HeLa-PD-1 cells were cultured in Dulbecco's modified Eagle's medium (Gibco) to which 10 % fetal bovine serum (Gibco) and 0.2 g/L hygromycin B were added in a 100 mm cell culture dish. HeLa-PD-1 cells cultured 24 hours before transfection were seeded in a 12-well plate. After 24 hours of the seeding, asiRNA (asiPD-1-93) was transfected using RNAimax (Invitrogen) based on the protocol provided by the manufacturer, and cp-asiPD-1-22 and cp-asiPD-1-23 were treated without a separate carrier. Thereafter, the PD-1 protein level was confirmed through Western blotting in the same manner as in Example 2.2 (FIG. 7). The experiment was independently conducted three times, and FIG. 7 shows the representative result.

As shown in FIG. 7, the gene expression inhibitory effect of No. 22, which was the most efficient, started to be seen at the protein level between days 2 and 3 after treatment, and the effect was maintained on day 4, after which the expression of the target gene PD-1 began to recover. Therefore, it was confirmed that the effect of cp-asiPD-1 at No. 22 maintained longer than that of asiPD-1 and cp-asiPD-1 at No. 23.

### Example 5: Functional assay for T cell activation ability of 2 types of cp-asiRNAs (cp-asiPD-1-22 and cp-asiPD-1-23)

It was confirmed whether the inhibition of PD-1 gene expression using two cp-asiRNAs (cp-asiPD-1-22 and cp-asiPD-1-23) shown in FIG. 7 led to an increase in T cell activity. Two types of cp-asiRNAs (cp-asiPD-1-22 and cp-asiPD-1-23) and asiPD-1-93 were used, and Nivolumab, a commercially available PD-1 blocking antibody, was also used to confirm the effect. cp-asiPIN1 was used as a negative control as a cp-asiRNA targeting a gene other than PD-1. The principle of the experiment is that when T cells recognize the antigen bound to the MHC molecule on the surface of the antigen-presenting cell through TCR, one of the results is that the expression of NFAT is promoted. When PD-1 is expressed, TCR interferes the signaling system, resulting in inhibition of NFAT expression. Therefore, PD-1/PD-L1 Blockade Bioassays (Promega) were used to quantitatively confirm the effect of activity inhibition of PD-1 or reducing of expression of PD-1 by using an effector cell (T cell) that has undergone genetic manipulation to express luciferase when NFAT is expressed.

### 5.1. Functional assay for T cell activation ability of two cp-asiRNAs (cp-asiPD-1-22 and cp-asiPD-1-23) (96 hours)

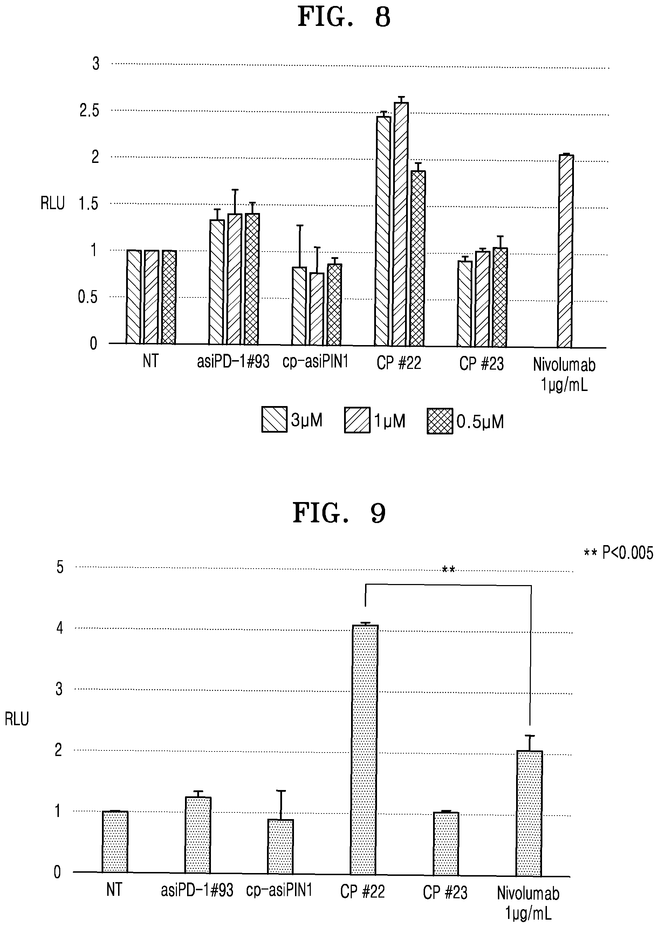

Specifically, effector cells expressing PD-1 were treated with asiPD-1-93 at concentrations of 3 µM, 1 µM, and 0.5 µM in the same manner as in Example 1.3, and cp-asiPD-1-22 and cp-asiPD-1-23 were treated at the same concentration in the same manner as in Example 2.2. After 96 hours after treatment, co-incubation with PD-L1 cells was performed for 6 hours, and then the luminescence was measured to determine the luminescence measurement value of each sample compared to the non-treated sample (NT) as an average relative light unit (RLU). This measurement was performed using PD-1/PD-L1 Blockade Bioassays (Promega) and was carried out according to the protocol provided by the manufacturer. Nivolumab used as a positive control was used at a concentration of 1 µg/mL. The experiment was independently conducted three times, and FIG. 8 shows the mean±standard deviation value of each experiment.

As shown in FIG. 8, in recovering T cell activity, cp-asiPD-1-22 was similar to or better than Nivolumab, a PD-1 antibody currently available on the market.

### 5.2. Functional assay for T cell activation ability of two cp-asiRNAs (cp-asiPD-1-22 and cp-asiPD-1-23) (72 hours)

As confirmed in Example 4 (FIG. 7), it was confirmed that the gene expression efficiency at the protein level of cp-asiPD-1-22 lasted from 3 days (72 hours) to 4 days (96 hours). As confirmed in Example 5.1 (FIG. 8), the T cell activation ability of cp-asiPD-1-22 was the most excellent, and it was confirmed that the T cell activity increased most at a concentration of 1 µM. Thus, the functional assay of the same method as in Example 5.1 was independently carried out for 72 hours three times. FIG. 9 is a graph showing the measured value of luminescence of each sample compared to the non-treated sample (NT) of the experiment as an average relative light unit (RLU).

As shown in FIG. 9, the effect of cp-asiPD-1 22 was about twice as good as the recovery of T cell activity caused by Nivolumab, and this difference is statistically significant, through p-value through T-test.

### Example 6: Screening of 8 RNAi-induced double-stranded nucleic acid molecules targeting PD-1

### 6.1. Design and production of 8 asiRNAs

In this Example, an RNAi-induced double-stranded nucleic acid molecule targeting PD-1 was designed and produced in the same manner as in Example 1.2. The nucleic acid molecule consisted of a sense strand of 16 nt and an antisense strand of 19 nt, and the screened 8 asiRNAs base sequences and target regions are shown in Table 5.

**[Table 5]**

| **asiRNA name** | **Seq uence(5' →3')** | | SEQ **ID** NO. |
|---|---|---|---|
| 8-039 | sense | CUAAACUGGUACCGCA | 77 |
| | antisense | UGCGGUACCAGUUUAGCAC | 78 |
| 8-046 | sense | GGAGAGCUUCGUGCUA | 79 |
| | antisense | UAGCACGAAGCUCUCCGAU | 80 |
| 8-048 | sense | CGGAGAGCUUCGUGCU | 81 |
| | antisense | AGCACGAAGCUCUCCGAUG | 82 |
| 8-050 | sense | UCUGGGCGGUGCUACA | 83 |
| | antisense | UGUAGCACCGCCCAGACGA | 84 |
| 8-053 | sense | CGCAGAUCAAAGAGAG | 85 |
| | antisense | CUCUCUUUGAUCUGCGCCU | 86 |
| 8-054 | sense | GCAGAUCAAAGAGAGC | 87 |
| | antisense | GCUCUCUUUGAUCUGCGCC | 88 |
| 8-068 | sense | GGAGUAUGCCACCAUU | 89 |
| | antisense | AAUGGUGGCAUACUCCGUC | 90 |
| 8-070 | sense | CCAUUGUCUUUCCUAG | 91 |
| | antisense | CUAGGAAAGACAAUGGUGG | 92 |

### 6.2. Confirmation of PD-1 mRNA expression inhibitory effect

In this example, to confirm the efficiency of gene inhibitory effect at the mRNA level, 8 types of asiRNAs were used to transfect a HeLa-PD-1 cell line at a concentration of 1 nM in the same manner as in Example 1.3, followed by performing qRT-PCR and measuring the PD-1 mRNA expression level. Specifically, 24 hours after seeding HeLa-PD-1 cells in a 12-well plate, asiRNA was transfected at a concentration of 1 nM using RNAimax (Thermo) according to a protocol provided by the manufacturer. After 24 hours of the transfection, RNA was extracted using Tri-RNA reagent (FAVORGEN), and cDNA was synthesized using High-capacity cDNA reverse transcription kit (Applied Biosystems). Thereafter, the efficiency of asiRNA to inhibit PD-1 expression was evaluated through qRT-PCR at the mRNA level.

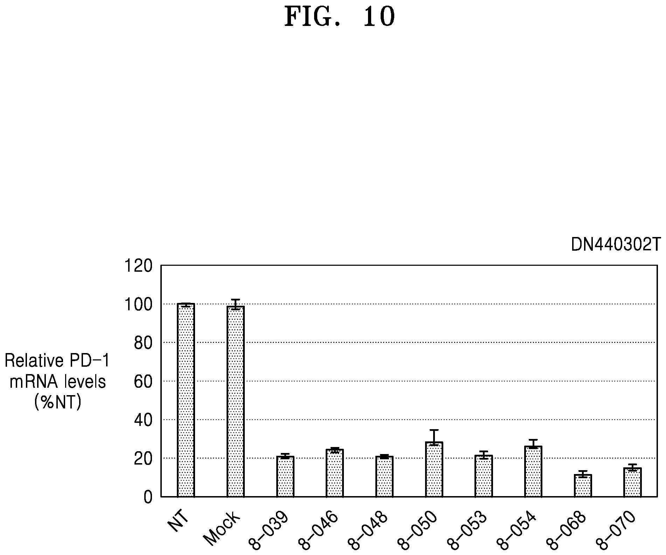

As a result, as shown in FIG. 10, it was confirmed that all of the RNAi-induced double-stranded nucleic acid molecules reduced the expression of PD-1 mRNA.

### 6.3. Confirmation of PD-1 protein expression inhibitory effect

In this Example, asiRNAs (8-039, 8-046, 8-048, 8-053, 8-068, and 8-070) showing a gene inhibitory efficiency of about 70 % or higher according to the results of Example 6.2 were transfected into the HeLa-PD-1 cell line, and then, the cells obtained 48 hours later were evaluated for the inhibitory efficiency of asiRNAs to PD-1 expression at the protein level in the same manner as in Example 1.4.

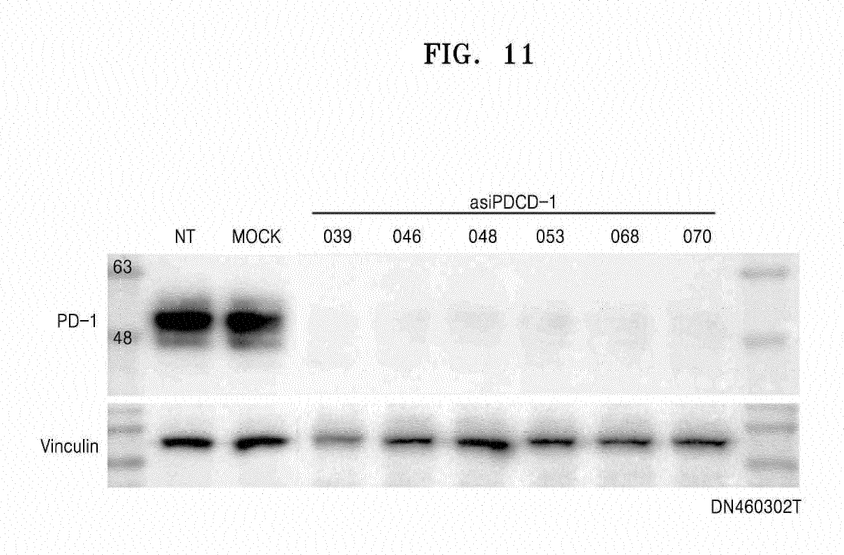

As a result, as shown in FIG. 11, it was confirmed that all of the RNAi-induced double-stranded nucleic acid molecules reduced the expression of PD-1 protein.

### Example 7: Screening of RNAi-induced asymmetric double-stranded nucleic acid molecules (cp-asiRNA) having cell-penetrating ability targeting PD-1

### 7.1. Design and production of 12 cp-asiRNAs of 8-039 and 8-068 further chemically modified

Chemical modifications were introduced in the same manner as in Example 3.1 for 8-068 and 8-039, which showed excellent PD-1 expression inhibitory efficiency among the 8 asiRNAs in Example 6. A total of 12 cp-asiRNAs were additionally designed and produced, and the nucleotide sequences thereof are shown in Table 6.

**[Table 6]**

| cp-asiRNA name | Sequence(5'→3') | |
|---|---|---|
| 8-068-2 | sense | GGAGmUAmUGmCmCAmCmCA*mU*mU*/chol/ |
| | antisense | 5'P-mAAmUGmGUmGGmCAmUAmCUmC*C*mG*U*mC |
| 8-068-3 | sense | GGAGmUAmUGmCmCAmCmCA*mU*mU*/chol/ |
| | antisense | 5'P-mA(2'F-A)mU(2'F-G)mG(2'F-U)mG(2'F-G)mC(2'F-A)mU(2'F- A)mC(2'F-U)mC*(2'F-C)*mG*(2'F-U)*mC |
| 8-068-5 | sense | GGAGmUAmUGmCmCAmCmCA*mU*mU*/chol/ |
| | antisense | 5'P-AAUGGUGGCAUACUmC*mC*mG*mU*mC |
| 8-068-7 | sense | mGGmAGmUAmUGmCCmACmCA*mU*U*/chol/ |
| | antisense | 5'P-mAAmUGmGUmGGmCAmUAmCUmC*C*mG*U*mC |
| 8-068-8 | sense | mGGmAGmUAmUGmCCmACmCA*mU*U*/chol/ |
| | antisense | 5'P-mA(2'F-A)mU(2'F-G)mG(2'F-U)mG(2'F-G)mC(2'F-A)mU(2'F- A)mC(2'F-U)mC*(2'F-C)*mG*(2'F-U)*mC |
| 8-068-13 | sense | mG(2'F-G)mA(2'F-G)mU(2'F-A)mU(2'F-G)mC(2'F-C)mA(2'F-C)mC(2'F- A)*mU*(2'F-U)* /chol/ |
| | antisense | 5'P-mA(2'F-A)mU(2'F-G)mG(2'F-U)mG(2'F-G)mC(2'F-A)mU(2'F- A)mC(2'F-U)mC*(2'F-C)*mG*(2'F-U)*mC |
| 8-068-15 | sense | mG(2'F-G)mA(2'F-G)mU(2'F-A)mU(2'F-G)mC(2'F-C)mA(2'F-C)mC(2'F- A)*mU*(2'F-U)*/chol/ |
| | antisense | 5'P-AAUGGUGGCAUACUmC*mC*mG*mU*mC |
| 8-039-3 | sense | mCmUAAAmCmUGGmUAmCmCG*mC*A*/chol/ |
| | antisense | 5'P-mU(2'-F-G)mC(2'-F-G)mG(2'-F-U)mA(2'-F-C)mC(2'-F-A)mG(2'-F- U)mU(2'-F-U)mA*(2'-F-G)*mC*(2'-F-A)*mC |
| 8-039-8 | sense | mCUmAAmACmUGmGUmACmCG*mC*A*/chol/ |
| | antisense | 5'P-mU(2'-F-G)mC(2'-F-G)mG(2'-F-U)mA(2'-F-C)mC(2'-F-A)mG(2'-F- U)mU(2'-F-U)mA*(2'-F-G)*mC*(2'-F-A)*mC |
| 8-039-13 | sense | mC(2'-F-U)mA(2'-F-A)mA(2'-F-C)mU(2'-F-G)mG(2'-F-U)mA(2'-F- C)mC(2'-F-G)*mC*(2'-F-A)*/chol/ |
| | antisense | 5'P-mU(2'-F-G)mC(2'-F-G)mG(2'-F-U)mA(2'-F-C)mC(2'-F-A)mG(2'-F- U)mU(2'-F-U)mA*(2'-F-G)*mC*(2'-F-A)*mC |
| 8-039-14 | sense | mC(2'-F-U)mA(2'-F-A)mA(2'-F-C)mU(2'-F-G)mG(2'-F-U)mA(2'-F- C)mC(2'-F-G)*mC*(2'-F-A)*/chol/ |
| | antisense | 5'P-(2'-F-U)mG(2'-F-C)mGmG(2'-F-U)mA(2'-F-C)(2'-F-C)mAmG(2'-F- U)(2'-F-U)(2'-F-U)mA*mG*(2'-F-C)*mA*(2'-F-C) |
| 8-039-15 | sense | mC(2'-F-U)mA(2'-F-A)mA(2'-F-C)mU(2'-F-G)mG(2'-F-U)mA(2'-F- C)mC(2'-F-G)*mC*(2'-F-A)*/chol/ |
| | antisense | 5'P-UGCGGUACCAGUUUmA*mG*mC*mA*mC |

### 7.2. Confirmation of PD-1 mRNA expression inhibitory effect

In this example, to confirm the efficiency of gene inhibitory effect at the mRNA level, 12 types of cp-asiRNAs were used to treat a HeLa-PD-1 cell line at a concentration of 1 µM, followed by performing qRT-PCR and measuring the PD-1 mRNA expression level. Specifically, 24 hours after seeding of HeLa-PD-1 cells in a 12-well plate, cp-asiRNA was treated without a separate carrier. After 24 hours of the treatment, RNA was extracted using Tri-RNA reagent (FAVORGEN), and cDNA was synthesized using High-capacity cDNA reverse transcription kit (Applied Biosystems). Thereafter, the efficiency of cp-asiRNA to inhibit PD-1 expression was evaluated through qRT-PCR at the mRNA level. 4 types, i.e., 8-068-3, 8-068-13, 8-039-8, and 8-039-13, selected from the 12 types of cp-asiRNAs were used to treat a HeLa-PD-1 cell line at a concentration of 1 µM or 2 µM, followed by evaluation of the PD-1 expression inhibitory efficiency at the mRNA level.

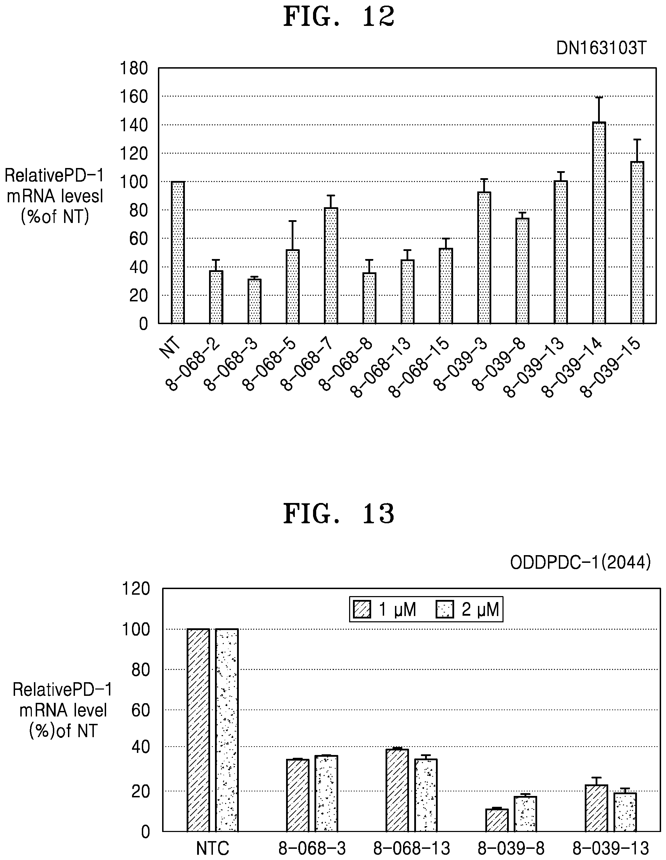

As a result, as shown in FIG. 12, 8-068-2, 8-068-3, 8-068-5, 8-068-7, 8-068-8, 8-068-13, 8-068-15, 8-039-3, and 8-039-13 from among the RNAi-induced double-stranded nucleic acid molecules having a cell-penetrating ability were found to reduce PD-1 mRNA expression. As shown in FIG. 13, 8-068-3, 8-068-13, 8-039-3, and 8-039-13 showed significant expression inhibitory effects.

### 7.3. Confirmation of PD-1 mRNA protein expression inhibitory effect

In this Example, 4 types of cp-asiRNAs showing excellent gene inhibitory efficiency according to the results of Example 7.1 were treated with HeLa-PD-1 cell lines, and then cells obtained after incubation for 24 hours were evaluated for the PD-1 expression inhibitory effects of cp-asiRNA at the protein level in the same manner as in Example 1.4.

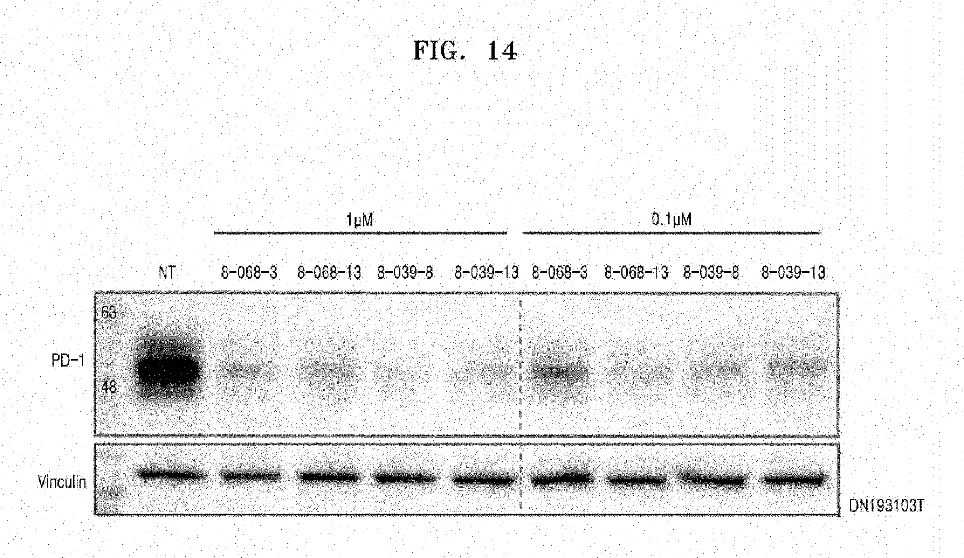

As a result, as shown in FIG. 14, all the RNAi-induced double-stranded nucleic acids (8-068-3, 8-068-13, 8-039-8, and 8-039-13) having a cell-penetrating ability were found to reduce PD-1 protein expression.

### Example 8: Functional assay for T cell activation ability of cp-asiRNAs

In this Example, T cells were treated with 8-068-3, 8-068-13, 8-039-8, and 8-039-13, which showed excellent PD-1 expression inhibitory efficiency among the cp-asiRNAs in Example 7, to confirm whether the inhibition of PD-1 gene expression leads to an increase in T cell activity. In particular, T cells were recovered for 18 to 20 hours by using frozen human T cells (available from Stem Cell Technologies). Then, the recovered human T cells were treated with CD3/Cd28 magnetic beads (Dynabeads) and 200 IU of IL-2 in CTS OpTmizer (Thermo) containing 2 % human serum to activate the T cells for 72 hours. After 72 hours of cp-asiRNA treatment on 20,000 activated T cells in a 12-well plate, RNA was extracted using Tri-RNA reagent (FAVORGEN), and cDNA was synthesized using High-capacity cDNA reverse transcription kit (Applied Biosystems). Thereafter, the efficiency of the T cells treated as above to inhibit PD-1 expression was evaluated through qRT-PCR at the mRNA level.

Then, using an effector cell (T cell) that has been genetically engineered to express luciferase, to quantitatively confirm the effect of inhibiting the activity or reducing the expression of PD-1, Promega bioassay kit (Catalogue No: J4011) was used to perform PD-1 blockade assay.

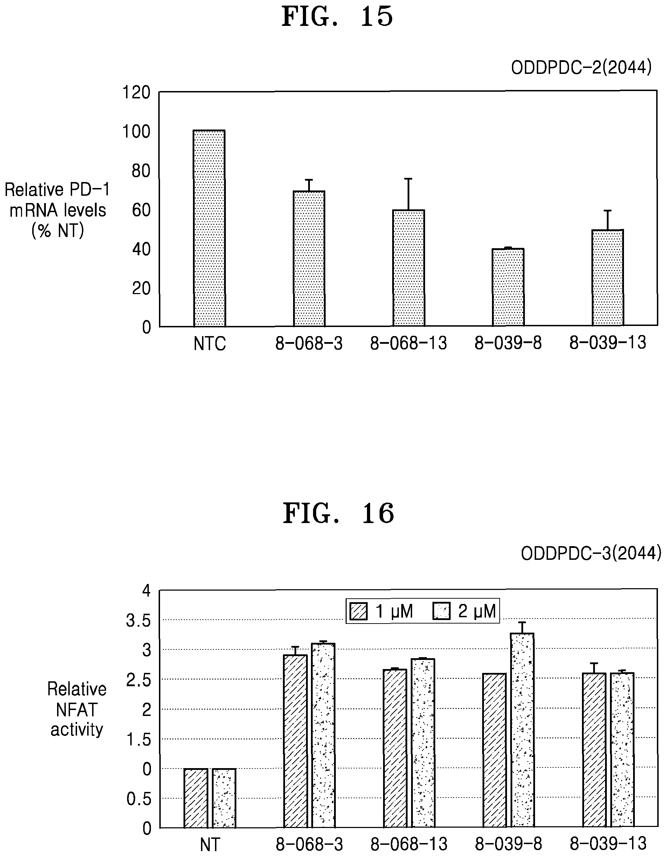

As a result, as shown in FIG. 15, all the RNAi-induced double-stranded nucleic acids (8-068-3, 8-068-13, 8-039-8, and 8-039-13) having a cell-penetrating ability were found to reduce PD-1 mRNA expression in T cells. In addition, as shown in FIG. 16, it was confirmed that the group treated with the RNAi-induced double-stranded nucleic acid molecule having a cell-penetrating ability, as compared with the recovery of T cell activity of the untreated group (NT), exhibited about three times more excellent expression inhibition efficiency.

As described above, specific parts of the present invention have been described in detail, and it will be apparent to of of ordinary skill in the art that this specific description is only a preferred embodiment, and the scope of the present invention is not limited thereto. Accordingly, it will be said that the substantial scope of the present invention is defined by the appended claims and their equivalents.

## Claims

1. An siRNA comprising: an antisense strand comprising a sequence complementary to mRNA encoding programmed cell death protein 1 (PD-1); a sense strand forming complementary bonds with the antisense strand, wherein a 5' end of the antisense strand and a 3' end of the sense strand form a blunt end.

2. The siRNA of claim 1, wherein the sense strand has a length of 15 nt to 17 nt, and the antisense strand has a length of 16 nt or more.

3. The siRNA of claim 2, wherein the antisense strand has a length of 16 nt to 31 nt.

4. The siRNA of claim 2, wherein the antisense strand has a length of 19 nt to 25 nt.

5. The siRNA of claim 1, wherein the sense strand is selected from the group consisting of SEQ ID NOs:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 77, 79, 81, 83, 85, 87, 89, and 91.

6. The siRNA of claim 5, wherein the sense strand is selected from the group consisting of SEQ ID NOs:43, 47, 49, 77, and 89.

7. The siRNA of claim 1, wherein the antisense strand is selected from the group consisting of SEQ ID NOs: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 78, 80, 82, 84, 86, 88, 90, and 92.

8. The siRNA of claim 7, wherein the antisense strand is selected from the group consisting of SEQ ID NOs:44, 48, 50, 78, and 90.

9. The siRNA of claim 1, wherein the sense strand or the antisense strand comprises at least one chemical modification selected from the group consisting of (a) to (e):
(a) an -OH group at a 2' carbon position of a sugar structure of a nucleotide substituted with -CH₃, -OCH₃, -NH₂, -F(fluorine), -O-2-methoxyethyl, -O-propyl, -O-2-methylthioethyl, -O-3-aminopropyl, or -O-3-dimethylaminopropyl;
(b) oxygen in a sugar structure of a nucleotide substituted with sulfur;
(c) a phosphate backbone of a nucleotide modified with phosphorothioate, boranophosphate, or methyl phosphonate;
(d) a nucleotide substituted with a peptide nucleic acid (PNA), a locked nucleic acid (LNA), or an unlocked nucleic acid(UNA); and
(e) a bonding of a phosphate group, a lipophilic compound, or a cell-penetrating peptide.

10. The siRNA of claim 9, wherein the lipophilic compound is selected from the group consisting of cholesterol, tocopherol, docosahexaenoic acid (DHA), palmitic acid, and long-chain fatty acids having 10 or more carbon atoms.

11. The siRNA of claim 9, comprising at least one chemical modification selected from the group consisting of: (a) an -OH group at at least one 2' carbon position of a sugar structure of a nucleotide substituted with -OCH₃ or -F in the sense strand or the antisense strand;
(b) at least one nucleotide bond in the sense strand or the antisense strand modified with a phosphorothioate bond;
(c) a cholesterol bonded to a 3' end of the sense strand; and
(d) phosphate group bonded to a 5' end of the antisense strand.

12. The siRNA of claim 11, wherein the sense strand is selected from the group consisting of (a) to (c) in a following table, and the antisense strand is selected from the group consisting of (d) to (o) in the following table:
| | **Sequence (5' →3')** |
|---|---|
| Sense | |
| (a) | mCAmGGmGUmGAmCAmGAmGA*MG*A*A/chol/ |
| (b) | mAGmGGmUGmACmAGmAGmAG*mA*A*/chol/ |
| (c) | mCAmUGmAGmCCmCCmAGmCA*mA*C*/chol/ |
| Antisense | |
| (d) | UCUCUCUGUCACCCmU*mG*A*G*C |
| (e) | UCUCUCUGUCACCCmU*mG*mA*mG*mC |
| (f) | UCUCUCUGUCACCCmUmGmAmGmCmUmC*mU*mG*mC*mC |
| (g) | UCUCUCUGUCACCCmUmGmAmGmCmUmC*mU*G*C*C |
| (h) | UCUCUCUGUCACCCmUmGmAmGmCmUC*U*G*C*C |
| (j) | OUCUCUCUGUCACCmC*mU*G*A*G |
| (j) | UUCUCUCUGUCACCmC*mU*mG*mA*mG |
| (k) | UUCUCUCUGUCACCmCmUmGmAmGmCmU*mC*mU*mG*mC |
| (l) | UUCUCUCUGUCACCmCmUmGmAmGmCmU*mC*U*G*C |
| (m) | UUCUCUCUGUCACCmCmUmGmAmGmCU*C*U*G*C |
| (n) | GUUGCUGGGGDUCAmU*mG*mC*mG*mG |
| (o) | GUUGCUGGGGCUCAmU*mG*C*G*G |
| In the sequences above, * indicates a phosphorothioated bond, m indicates 2'-O-methyl, and /chol/ indicates cholesterol. | |

13. The siRNA of claim 12, wherein the sense strand is (b) in the table, the antisense strand is at least one selected from the group consisting of (i) to (m) in the table, and a 5' end of the antisense strand is bound to a phosphate group.

14. The siRNA of claim 11, wherein the sense strand is selected from the group consisting of (a) to (c) in a following table, and the antisense strand is selected from the group consisting of (d) to (h) in the following table:
| | Sequence (5'→3') |
|---|---|
| Sense | |
| (a) | mAGmGGmUGmACmAGmAGmAG*mA*A*/chol/ |
| (b) | mA(2'-F-G)mG(2'-F-G)mU(2'-F-G)mA(2'-F-C)mA(2'-F-G)mA(2'-F-G)mA(2'- F-G)*mA*(2'-F-A)*/chol/ |
| (c) | mAmGmGGmUGAmCAGAGAmG*mA*mA*/chol/ |
| Antisen se | |
| (d) | 5' P-UUCUCUCUGUCACCmCmUmGmAmGmCU*C*U*G*C |
| (e) | 5' P-mU*(2'-F-U)*mC(2'-F-U)mC(2'-F-U)mC(2'-F-U)mG(2'-F-U)mC(2'-F-A) mC(2'-F-C)mC(2'-F-U)mG(2'-F-A)mG(2'-F-C)mU*(2'-F-C)*mU*(2'-F-G)*mC |
| (f) | 5' P-mU*(2'-F-U)*(2'-F-C)(2'-F-U)(2'-F-C)(2'-F-U)(2'-F-C)(2'-F-U)G(2'-F-U) (2'-F-C)A(2'-F-C)CCUmGmAmGmCU*C*U*G*C |
| (g) | 5' P-UCUCUCUGUCACCCmUmGmAmGmCmUmC*mU*mG*mC*mC |
| (h) | 5' P-UCUCUCUGUCACCCmU*mG*rnA*mG*mC |
| In the sequences above, * indicates a phosphorothioated bond, m indicates 2'-O-methyl, 2'-F- indicates 2'-F(fluorine), /chol/ indicates cholesterol, and 5'P indicates a 5'-phosphate group. | |

15. The siRNA of claim 14, wherein the sense strand is (c) in the table, the antisense strand is (g) or (h) in the table.

16. The siRNA of claim 11, wherein the sense strand is selected from the group consisting of (A) to (D) in a following table, and the antisense strand is selected from the group consisting of (E) to (H) in the following table:
| | **Sequence (5' →3')** |
|---|---|
| Sense | |
| (A) | GGAGmUAmUGmCmCAmCmCA*mU*mU*/chol/ |
| (B) | mG(2'F-G)mA(2'F-G)mU(2'F-A)mU(2'F-G)mC(2'F-C)mA(2'F-C)mC(2'F-A) *mU*(2'F-U)*/chol/ |
| (C) | mCUmAAmACmUGmGUmACmCG*mC*A*/chol/ |
| (D) | mC(2'-F-U)mA(2'-F-A)mA(2'-F-C)mU(2'-F-G)mG(2'-F-U)mA(2'-F-C)mC (2'-F-G)*mC*(2'-F-A)*/chol/ |
| Antisense | |
| (E) | 5'P-mA(2'F-A)mU(2'F-G)mG(2'F-U)mG(2'F-G)mC(2'F-A)mU(2'F-A)mC(2'F-U) mC*(2'F-C)*mG*(2'F-U)*mC |
| (F) | 5'P-mA(2'F-A)mU(2'F-G)mG(2'F-U)mG(2'F-G)mC(2'F-A)mU(2'F-A)mC(2'F-U) mC*(2'F-C)*mG*(2'F-U)*mC |
| (G) | 5'P-mU(2'-F-G)mC(2'-F-G)mG(2'-F-U)mA(2'-F-C)mC(2'-F-A)mG(2'-F-U) mU(2'-F-U)mA*(2'-F-G)*mC*(2'-F-A)*mC |
| (H) | 5'P-mU(2'-F-G)mC(2'-F-G)mG(2'-F-U)mA(2'-F-C)mC(2'-F-A)mG(2'-F-U) mU(2'-F-U)mA*(2'-F-G)*mC*(2'-F-A)*mC |
| In the sequences above, * indicates a phosphorothioated bond, m indicates 2'-methyl, 2'-F-indicates 2'-F(fluorine), /chol/ indicates cholesterol, and 5'P indicates a 5'-phosphate group. | |

17. A pharmaceutical composition for preventing or treating cancer, comprising the siRNA according to any one of claims 1 to 16.

18. The pharmaceutical composition of claim 17, wherein the cancer is selected from multiple myeloma, non-epithelial tumor, non-small cell lung cancer (NSCLC), melanoma, breast cancer, glioma, lymphoma, leukemia, urinary system cancer, digestive tract cancer, reproductive system tumor, refractory classic Hodgkin's lymphoma, prostate cancer, metastatic melanoma, renal cell carcinoma, Hodgkin lymphoma, squamous cell carcinoma of the head and neck, urinary tract epithelial cell carcinoma, refractory B cell precursor acute lymphocytic leukemia, and diffuse large B cell lymphoma.

19. An immune cell in which expression of PD-1 is suppressed, obtained by treating the immune cell with the siRNA according to any one of claims 1 to 16.

20. The immune cell of claim 19, wherein the immune cell is selected from the group consisting of a natural killer cell (NK cell), a chimeric antigen receptor-modified NK cell (CAR-NK cell), a universal-NK cell, a tumor-infiltrating T lymphocytle (TIL), a peripheral blood mononuclear cell (PBMC), a T receptor-modified T cell (TCR-T), and a chimeric antigen receptor-modified T cell (CAR-T).

21. An immune cell therapeutic agent for treating cancer, comprising the immune cell according to claim 19 in which expression of PD-1 is suppressed.


<110>    OliX Pharmaceuticals, Inc.

<120>    Asymmetric siRNA Inhibiting Expression of PD-1

<130>    PX061980

<150>    KR 2019-0058805
<151>    2019-05-20

<160>    92

<170>    KoPatentIn 3.0

<210>    1
<211>    16
<212>    RNA
<213>    Artificial Sequence

<220>
<223>    siRNA


<400>    1
cuguggggcc aucucc                                                        16


<210>    2
<211>    21
<212>    RNA
<213>    Artificial Sequence

<220>
<223>    siRNA


<400>    2
ggagauggcc ccacagaggu a                                                  21


<210>    3
<211>    16
<212>    RNA
<213>    Artificial Sequence

<220>
<223>    siRNA


<400>    3
ucuguggggc caucuc                                                        16


<210>    4
<211>    21
<212>    RNA
<213>    Artificial Sequence

<220>
<223>    siRNA


<400>    4
gagauggccc cacagaggua g                                                  21

<210>    5
<211>    16
<212>    RNA
<213>    Artificial Sequence

<220>
<223>    siRNA


<400>    5
ccuucaccug cagcuu                                                    16


<210>    6
<211>    21
<212>    RNA
<213>    Artificial Sequence

<220>
<223>    siRNA


<400>    6
aagcugcagg ugaagguggc g                                              21


<210>    7
<211>    16
<212>    RNA
<213>    Artificial Sequence

<220>
<223>    siRNA


<400>    7
accuucaccu gcagcu                                                    16


<210>    8
<211>    21
<212>    RNA
<213>    Artificial Sequence

<220>
<223>    siRNA


<400>    8
agcugcaggu gaagguggcg u                                              21


<210>    9
<211>    16
<212>    RNA
<213>    Artificial Sequence

<220>
<223>    siRNA

<400>    9
caccuucacc ugcagc                                                    16


<210>    10
<211>    21
<212>    RNA
<213>    Artificial Sequence

<220>
<223>    siRNA


<400>    10
gcugcaggug aagguggcgu u                                              21


<210>    11
<211>    16
<212>    RNA
<213>    Artificial Sequence

<220>
<223>    siRNA


<400>    11
   cucugugggg ccaucu                                                     16


<210>    12
<211>    21
<212>    RNA
<213>    Artificial Sequence

<220>
<223>    siRNA


<400>    12
agauggcccc acagagguag g                                              21


<210>    13
<211>    16
<212>    RNA
<213>    Artificial Sequence

<220>
<223>    siRNA


<400>    13
ccucuguggg gccauc                                                    16


<210>    14
<211>    21
<212>    RNA
<213>    Artificial Sequence

<220>
<223>    siRNA

<400>    14
gauggcccca cagagguagg u                                                21

<210>    15
<211>    16
<212>    RNA
<213>    Artificial Sequence

<220>
<223>    siRNA

<400>    15
accucugugg ggccau                                                      16

<210>    16
<211>    21
<212>    RNA
<213>    Artificial Sequence

<220>
<223>    siRNA

<400>    16
auggccccac agagguaggu g                                                21

<210>    17
<211>    16
<212>    RNA
<213>    Artificial Sequence

<220>
<223>    siRNA

<400>    17
ccaccuucac cugcag                                                      16

<210>    18
<211>    21
<212>    RNA
<213>    Artificial Sequence

<220>
<223>    siRNA

<400>    18
cugcagguga agguggcguu g                                                21

<210>    19
<211>    16
<212>    RNA
<213>    Artificial Sequence

<220>
<223>    siRNA


<400>    19
gccccagcaa ccagac                                                        16


<210>    20
<211>    21
<212>    RNA
<213>    Artificial Sequence

<220>
<223>    siRNA


<400>    20
gucugguugc uggggcucau g                                                  21


<210>    21
<211>    16
<212>    RNA
<213>    Artificial Sequence

<220>
<223>    siRNA


<400>    21
agccccagca accaga                                                        16


<210>    22
<211>    21
<212>    RNA
<213>    Artificial Sequence

<220>
<223>    siRNA


<400>    22
ucugguugcu ggggcucaug c                                                  21


<210>    23
<211>    16
<212>    RNA
<213>    Artificial Sequence

<220>
<223>    siRNA


<400>    23
ggcaccuacc ucugug                                                        16


<210>    24
<211>    21
<212>    RNA

<213>    Artificial Sequence

<220>
<223>    siRNA


<400>    24
cacagaggua ggugccgcug u                                                21


<210>    25
<211>    16
<212>    RNA
<213>    Artificial Sequence

<220>
<223>    siRNA


<400>    25
cggcaccuac cucugu                                                      16


<210>    26
<211>    21
<212>    RNA
<213>    Artificial Sequence

<220>
<223>    siRNA


<400>    26
acagagguag gugccgcugu c                                                21


<210>    27
<211>    16
<212>    RNA
<213>    Artificial Sequence

<220>
<223>    siRNA


<400>    27
gcggcaccua ccucug                                                      16


<210>    28
<211>    21
<212>    RNA
<213>    Artificial Sequence

<220>
<223>    siRNA


<400>    28
cagagguagg ugccgcuguc a                                                21


<210>    29

<211>    16
<212>    RNA
<213>    Artificial Sequence

<220>
<223>    siRNA


<400>    29
agcggcaccu accucu                                                        16


<210>    30
<211>    21
<212>    RNA
<213>    Artificial Sequence

<220>
<223>    siRNA


<400>    30
agagguaggu gccgcuguca u                                                  21


<210>    31
<211>    16
<212>    RNA
<213>    Artificial Sequence

<220>
<223>    siRNA


<400>    31
cgugacuucc acauga                                                        16


<210>    32
<211>    21
<212>    RNA
<213>    Artificial Sequence

<220>
<223>    siRNA


<400>    32
ucauguggaa gucacgcccg u                                                  21


<210>    33
<211>    16
<212>    RNA
<213>    Artificial Sequence

<220>
<223>    siRNA


<400>    33
gggcgugacu uccaca                                                        16

<210>    34
<211>    21
<212>    RNA
<213>    Artificial Sequence

<220>
<223>    siRNA


<400>    34
uguggaaguc acgcccguug g                                                21


<210>    35
<211>    16
<212>    RNA
<213>    Artificial Sequence

<220>
<223>    siRNA


<400>    35
acuaugggga gcugga                                                      16


<210>    36
<211>    21
<212>    RNA
<213>    Artificial Sequence

<220>
<223>    siRNA


<400>    36
uccagcuccc cauaguccac a                                                21


<210>    37
<211>    16
<212>    RNA
<213>    Artificial Sequence

<220>
<223>    siRNA


<400>    37
gacacugcuc uuggcc                                                      16


<210>    38
<211>    21
<212>    RNA
<213>    Artificial Sequence

<220>
<223>    siRNA


<400>    38

ggccaagagc aguguccauc c                                                21

<210>    39
<211>    16
<212>    RNA
<213>    Artificial Sequence

<220>
<223>    siRNA

<400>    39
gccaccuuca ccugca                                                      16

<210>    40
<211>    21
<212>    RNA
<213>    Artificial Sequence

<220>
<223>    siRNA

<400>    40
ugcaggugaa gguggcguug u                                                21

<210>    41
<211>    16
<212>    RNA
<213>    Artificial Sequence

<220>
<223>    siRNA

<400>    41
cgccaccuuc accugc                                                      16

<210>    42
<211>    21
<212>    RNA
<213>    Artificial Sequence

<220>
<223>    siRNA

<400>    42
gcaggugaag guggcguugu c                                                21

<210>    43
<211>    16
<212>    RNA
<213>    Artificial Sequence

<220>
<223>    siRNA

<400>    43
caugagcccc agcaac                                                        16


<210>    44
<211>    21
<212>    RNA
<213>    Artificial Sequence

<220>
<223>    siRNA


<400>    44
guugcugggg cucaugcggu a                                                  21


<210>    45
<211>    16
<212>    RNA
<213>    Artificial Sequence

<220>
<223>    siRNA


<400>    45
gcaugagccc cagcaa                                                        16


<210>    46
<211>    21
<212>    RNA
<213>    Artificial Sequence

<220>
<223>    siRNA


<400>    46
uugcuggggc ucaugcggua c                                                  21


<210>    47
<211>    16
<212>    RNA
<213>    Artificial Sequence

<220>
<223>    siRNA


<400>    47
agggugacag agagaa                                                        16


<210>    48
<211>    21
<212>    RNA
<213>    Artificial Sequence

<220>

<223>    siRNA

<400>    48
uucucucugu cacccugagc u                                                21

<210>    49
<211>    16
<212>    RNA
<213>    Artificial Sequence

<220>
<223>    siRNA

<400>    49
cagggugaca gagaga                                                      16

<210>    50
<211>    21
<212>    RNA
<213>    Artificial Sequence

<220>
<223>    siRNA

<400>    50
ucucucuguc acccugagcu c                                                21

<210>    51
<211>    16
<212>    RNA
<213>    Artificial Sequence

<220>
<223>    siRNA

<400>    51
ucagggugac agagag                                                      16

<210>    52
<211>    21
<212>    RNA
<213>    Artificial Sequence

<220>
<223>    siRNA

<400>    52
cucucuguca cccugagcuc u                                                21

<210>    53
<211>    16
<212>    RNA
<213>    Artificial Sequence

<220>
<223>    siRNA

<400>    53
gacuaugggg agcugg                                                    16

<210>    54
<211>    21
<212>    RNA
<213>    Artificial Sequence

<220>
<223>    siRNA

<400>    54
ccagcucccc auaguccaca g                                              21

<210>    55
<211>    16
<212>    RNA
<213>    Artificial Sequence

<220>
<223>    siRNA

<400>    55
aguggcgaga gaagac                                                    16

<210>    56
<211>    21
<212>    RNA
<213>    Artificial Sequence

<220>
<223>    siRNA

<400>    56
gucuucucuc gccacuggaa a                                              21

<210>    57
<211>    16
<212>    RNA
<213>    Artificial Sequence

<220>
<223>    siRNA

<400>    57
caguggcgag agaaga                                                    16

<210>    58
<211>    21

<212>    RNA
<213>    Artificial Sequence

<220>
<223>    siRNA


<400>    58
ucuucucucg ccacuggaaa u                                                21


<210>    59
<211>    16
<212>    RNA
<213>    Artificial Sequence

<220>
<223>    siRNA


<400>    59
ccaguggcga gagaag                                                      16


<210>    60
<211>    21
<212>    RNA
<213>    Artificial Sequence

<220>
<223>    siRNA


<400>    60
cuucucucgc cacuggaaau c                                                21


<210>    61
<211>    16
<212>    RNA
<213>    Artificial Sequence

<220>
<223>    siRNA


<400>    61
uccaguggcg agagaa                                                      16


<210>    62
<211>    21
<212>    RNA
<213>    Artificial Sequence

<220>
<223>    siRNA


<400>    62
uucucucgcc acuggaaauc c                                                21

<210>    63
<211>    16
<212>    RNA
<213>    Artificial Sequence

<220>
<223>    siRNA


<400>    63
ucugcagacc cuccac                                                        16


<210>    64
<211>    21
<212>    RNA
<213>    Artificial Sequence

<220>
<223>    siRNA


<400>    64
guggaggguc ugcagaacac u                                                  21


<210>    65
<211>    21
<212>    RNA
<213>    Artificial Sequence

<220>
<223>    siRNA


<400>    65
gcctggctcc tattgtccct c                                                  21


<210>    66
<211>    21
<212>    RNA
<213>    Artificial Sequence

<220>
<223>    siRNA


<400>    66
caggugaagg uggcguuguc c                                                  21


<210>    67
<211>    16
<212>    RNA
<213>    Artificial Sequence

<220>
<223>    siRNA


<400>    67
aacgccaccu ucaccu                                                        16

<210>    68
<211>    21
<212>    RNA
<213>    Artificial Sequence

<220>
<223>    siRNA


<400>    68
aggugaaggu ggcguugucc c                                                  21


<210>    69
<211>    16
<212>    RNA
<213>    Artificial Sequence

<220>
<223>    siRNA


<400>    69
uucugcagac ccucca                                                        16


<210>    70
<211>    21
<212>    RNA
<213>    Artificial Sequence

<220>
<223>    siRNA


<400>    70
uggagggucu gcagaacacu g                                                  21


<210>    71
<211>    16
<212>    RNA
<213>    Artificial Sequence

<220>
<223>    siRNA


<400>    71
ggacuauggg gagcug                                                        16


<210>    72
<211>    21
<212>    RNA
<213>    Artificial Sequence

<220>
<223>    siRNA

<400>    72
cagcucccca uaguccacag a                                                21


<210>    73
<211>    20
<212>    DNA
<213>    Artificial Sequence

<220>
<223>    Foward Primer (Housekeeping gene Tublin)


<400>    73
gaccaagcgt accatccagt                                                  20


<210>    74
<211>    19
<212>    DNA
<213>    Artificial Sequence

<220>
<223>    Reverse Primer (Housekeeping gene Tublin)


<400>    74
acgtttggca tacatcagg                                                   19


<210>    75
<211>    19
<212>    DNA
<213>    Artificial Sequence

<220>
<223>    Foward Primer (PD-1 mRNA qPCR primer)


<400>    75
ccctggtggt tggtgtcgt                                                   19


<210>    76
<211>    21
<212>    DNA
<213>    Artificial Sequence

<220>
<223>    Reverse Primer (PD-1 mRNA qPCR primer)


<400>    76
gcctggctcc tattgtccct c                                                21


<210>    77
<211>    16
<212>    RNA
<213>    Artificial Sequence

<220>
<223>    siRNA

<400>    77
cuaaacuggu accgca                                                    16


<210>    78
<211>    19
<212>    RNA
<213>    Artificial Sequence

<220>
<223>    siRNA


<400>    78
ugcgguacca guuuagcac                                                 19


<210>    79
<211>    16
<212>    RNA
<213>    Artificial Sequence

<220>
<223>    siRNA


<400>    79
ggagagcuuc gugcua                                                    16


<210>    80
<211>    19
<212>    RNA
<213>    Artificial Sequence

<220>
<223>    siRNA


<400>    80
uagcacgaag cucuccgau                                                 19


<210>    81
<211>    16
<212>    RNA
<213>    Artificial Sequence

<220>
<223>    siRNA


<400>    81
cggagagcuu cgugcu                                                    16


<210>    82
<211>    19
<212>    RNA
<213>    Artificial Sequence

<220>
<223>    siRNA


<400>    82
agcacgaagc ucuccgaug                                                    19


<210>    83
<211>    16
<212>    RNA
<213>    Artificial Sequence

<220>
<223>    siRNA


<400>    83
ucugggcggu gcuaca                                                       16


<210>    84
<211>    19
<212>    RNA
<213>    Artificial Sequence

<220>
<223>    siRNA


<400>    84
uguagcaccg cccagacga                                                    19


<210>    85
<211>    16
<212>    RNA
<213>    Artificial Sequence

<220>
<223>    siRNA


<400>    85
cgcagaucaa agagag                                                       16


<210>    86
<211>    19
<212>    RNA
<213>    Artificial Sequence

<220>
<223>    siRNA


<400>    86
cucucuuuga ucugcgccu                                                    19


<210>    87
<211>    16
<212>    RNA

<213>    Artificial Sequence

<220>
<223>    siRNA


<400>    87
gcagaucaaa gagagc                                                    16


<210>    88
<211>    19
<212>    RNA
<213>    Artificial Sequence

<220>
<223>    siRNA


<400>    88
gcucucuuug aucugcgcc                                                 19


<210>    89
<211>    16
<212>    RNA
<213>    Artificial Sequence

<220>
<223>    siRNA


<400>    89
ggaguaugcc accauu                                                    16


<210>    90
<211>    19
<212>    RNA
<213>    Artificial Sequence

<220>
<223>    siRNA


<400>    90
   aaugguggca uacuccguc                                                  19


<210>    91
<211>    16
<212>    RNA
<213>    Artificial Sequence

<220>
<223>    siRNA


<400>    91
ccauugucuu uccuag                                                    16


<210>    92

<211> 19
<212> RNA
<213> Artificial Sequence

<220>
<223> siRNA

<400> 92
cuaggaaaga caauggugg 19